(19) Europäisches Patentamt
European Patent Office
Office européen des brevets

(11) EP 3 234 064 B1

(12) **EUROPEAN PATENT SPECIFICATION**

(45) Date of publication and mention of the grant of the patent:
**27.02.2019 Bulletin 2019/09**

(21) Application number: **15798330.5**

(22) Date of filing: **23.11.2015**

(51) Int Cl.:
***C09K 19/02*** (2006.01) ***C09K 19/04*** (2006.01)
***C09K 19/14*** (2006.01) ***C09K 19/28*** (2006.01)
***C09K 19/20*** (2006.01) ***C09K 19/58*** (2006.01)
***C07C 315/04*** (2006.01) ***C07C 317/14*** (2006.01)
***C07C 45/68*** (2006.01) ***C07C 49/813*** (2006.01)
***C07C 17/26*** (2006.01) ***C07C 25/18*** (2006.01)

(86) International application number:
**PCT/EP2015/002349**

(87) International publication number:
**WO 2016/096081 (23.06.2016 Gazette 2016/25)**

(54) **BIMESOGENIC COMPOUNDS AND MESOGENIC MEDIA**

BIMESOGENE VERBINDUNGEN UND MESOGENE MEDIEN

COMPOSÉS BIMÉSOGÈNES ET MILIEUX MÉSOGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2014 EP 14004311**

(43) Date of publication of application:
**25.10.2017 Bulletin 2017/43**

(73) Proprietor: **Merck Patent GmbH**
**64293 Darmstadt (DE)**

(72) Inventors:
- **ADLEM, Kevin**
**Bournemouth BH8 9LS (GB)**
- **SAXTON, Patricia, Eileen**
**Salisbury SP5 2BE (GB)**

(56) References cited:
**WO-A1-2006/061094 WO-A1-2014/005672**

- **MAHAJAN R ET AL: "NEW MESOGENS WITH A FLUORO-SUBSTITUTED BENT CORE", LIQUID CRYSTALS, TAYLOR & FRANCIS, vol. 31, no. 2, 1 February 2004 (2004-02-01), pages 161-167, XP001047703, ISSN: 0267-8292, DOI: 10.1080/02678290310001599215**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 234 064 B1

## Description

The invention relates to a mesogenic medium comprising a bimesogenic compound of formula I

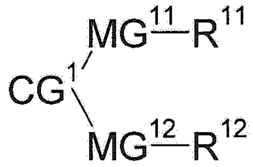

I

wherein $R^{11}$, $R^{12}$, $MG^{11}$, $MG^{12}$ and $CG^1$ have the meaning given herein below, to the use of the liquid crystal media in a liquid crystal device and in particular to flexoelectric liquid crystal devices comprising a liquid crystal medium according to the present invention.

Liquid Crystal Displays (LCDs) are widely used to display information. LCDs are used for direct view displays, as well as for projection type displays. The electro-optical mode which is employed for most displays still is the twisted nematic (TN)-mode with its various modifications. Besides this mode, the super twisted nematic (STN)-mode and more recently the optically compensated bend (OCB)-mode and the electrically controlled birefringence (ECB)-mode with their various modifications, as e. g. the vertically aligned nematic (VAN), the patterned ITO vertically aligned nematic (PVA)-, the polymer stabilized vertically aligned nematic (PSVA)-mode and the multi domain vertically aligned nematic (MVA)-mode, as well as others, have been increasingly used. All these modes use an electrical field, which is substantially perpendicular to the substrates, respectively to the liquid crystal layer. Besides these modes there are also electro-optical modes employing an electrical field substantially parallel to the substrates, respectively the liquid crystal layer, like e.g. the In Plane Switching (short IPS) mode (as disclosed e.g. in DE 40 00 451 and EP 0 588 568) and the Fringe Field Switching (FFS) mode. Especially the latter mentioned electro-optical modes, which have good viewing angle properties and improved response times, are increasingly used for LCDs for modern desktop monitors and even for displays for TV and for multimedia applications and thus are competing with the TN-LCDs.

Further to these displays, new display modes using cholesteric liquid crystals having a relatively short cholesteric pitch have been proposed for use in displays exploiting the so called "flexo-electric" effect. The term "liquid crystal", "mesomorphic compound" or "mesogenic compound" (also shortly referred to as "mesogen") means a compound that under suitable conditions of temperature, pressure and concentration can exist as a mesophase (nematic, smectic, etc.) or in particular as a LC phase. Non-amphiphilic mesogenic compounds comprise for example one or more calamitic, banana-shaped or discotic mesogenic groups.

Flexoelectric liquid crystal materials are known in prior art. The flexoelectric effect is described inter alia by Chandrasekhar, "Liquid Crystals", 2nd edition, Cambridge University Press (1992) and P.G. deGennes et al., "The Physics of Liquid Crystals", 2nd edition, Oxford Science Publications (1995).

In these displays the cholesteric liquid crystals are oriented in the "uniformly lying helix" arrangement (ULH), which also give this display mode its name. For this purpose, a chiral substance which is mixed with a nematic material induces a helical twist transforming the material into a chiral nematic material, which is equivalent to a cholesteric material. The term "chiral" in general is used to describe an object that is non-superimposable on its mirror image. "Achiral" (non-chiral) objects are objects that are identical to their mirror image. The terms chiral nematic and cholesteric are used synonymously herein, unless explicitly stated otherwise. The pitch induced by the chiral substance ($P_0$) is in a first approximation inversely proportional to the concentration (c) of the chiral material used. The constant of proportionality of this relation is called the helical twisting power (HTP) of the chiral substance and defined by equation (1)

$$HTP \equiv 1 / (c \cdot P_0) \qquad (1)$$

wherein

c is concentration of the chiral compound.

The uniform lying helix texture is realized using a chiral nematic liquid crystal with a short pitch, typically in the range from 0.2 μm to 1 μm, preferably of 1.0 μm or less, in particular of 0.5 μm or less, which is unidirectional aligned with its helical axis parallel to the substrates, e. g. glass plates, of a liquid crystal cell. In this configuration the helical axis of the chiral nematic liquid crystal is equivalent to the optical axis of a birefringent plate.

If an electrical field is applied to this configuration normal to the helical axis the optical axis is rotated in the plane of the cell, similar as the director of a ferroelectric liquid crystal rotates in a surface stabilized ferroelectric liquid

crystal display. The flexoelectric effect is characterized by fast response times typically ranging from 6 μs to 100 μs. It further features excellent grey scale capability.

The field induces a splay bend structure in the director which is accommodated by a tilt in the optical axis. The angle of the rotation of the axis is in first approximation directly and linearly proportional to the strength of the electrical field. The optical effect is best seen when the liquid crystal cell is placed between crossed polarisers with the optical axis in the unpowered state at an angle of 22.5° to the absorption axis of one of the polarisers. This angle of 22.5° is also the ideal angle of rotation of the electric field, as thus, by the inversion the electrical field, the optical axis is rotated by 45° and by appropriate selection of the relative orientations of the preferred direction of the axis of the helix, the absorption axis of the polarizer and the direction of the electric field, the optical axis can be switched from parallel to one polarizer to the centre angle between both polarisers. The optimum contrast is then achieved when the total angle of the switching of the optical axis is 45°. In that case the arrangement can be used as a switchable quarter wave plate, provided the optical retardation, i. e. the product of the effective birefringence of the liquid crystal and the cell gap, is selected to be the quarter of the wave length. In this context the wavelength referred to is 550 nm, the wavelength for which the sensitivity of the human eye is highest, unless explicitly stated otherwise.

The angle of rotation of the optical axis ($\Phi$) is given in good approximation by formula (2)

$$\tan \Phi = \bar{e}\, P_0\, E / (2 \pi K) \qquad (2)$$

wherein

- $P_0$ is the undisturbed pitch of the cholesteric liquid crystal,
- $\bar{e}$ is the average [$\bar{e} = ½ (e_{splay} + e_{bend})$] of the splay flexoelectric coefficient ($e_{splay}$) and the bend flexoelectric coefficient ($e_{bend}$),
- E is the electrical field strength and
- $K$ is the average [$K = ½ (k_{11} + k_{33})$] of the splay elastic constant ($k_{11}$) and the bend elastic constant ($K_{33}$)

and wherein
$\bar{e} / K$ is called the flexo-elastic ratio.

This angle of rotation is half the switching angle in a flexoelectric switching element.

The response time ($\tau$) of this electro-optical effect is given in good approximation by formula (3)

$$\tau = [P_0/(2 \pi)]^2 \cdot \gamma / K \qquad (3)$$

wherein

- $\gamma$ is the effective viscosity coefficient associated with the distortion of the helix.

There is a critical field ($E_c$) to unwind the helix, which can be obtained from equation (4)

$$E_c = (\pi^2 / P_0) \cdot [k_{22}/(\varepsilon_0 \cdot \Delta\varepsilon)]^{1/2} \qquad (4)$$

wherein

- $k_{22}$ is the twist elastic constant,
- $\varepsilon_0$ is the permittivity of vacuum and
- $\Delta\varepsilon$ is the dielectric anisotropy of the liquid crystal.

In this mode, however several problems still have to be resolved, which are, amongst others, difficulties in obtaining the required uniform orientation, an unfavourably high voltage required for addressing, which is incompatible with common driving electronics, a not really dark "off state", which deteriorates the contrast, and, last not least, a pronounced hysteresis in the electro-optical characteristics.

A relatively new display mode, the so-called uniformly standing helix (USH) mode, may be considered as an alternative mode to succeed the IPS, as it can show improved black levels, even compared to other display mode providing wide viewing angles (e.g. IPS, VA etc.).

For the USH mode, like for the ULH mode, flexoelectric switching has been proposed, using bimesogenic liquid crystal materials. Bimesogenic compounds are known in general from prior art (cf. also Hori, K., limuro, M., Nakao, A., Toriumi, H., J. Mol. Struc. 2004, 699, 23-29). The term "bimesogenic compound" relates to compounds comprising two mesogenic groups in the molecule. Just like normal mesogens they can form many mesophases, depending on their structure. In particular compounds of formula I induce a second nematic phase, when added to a nematic liquid crystal medium.

The term "mesogenic group" means in this context, a group with the ability to induce liquid crystal (LC) phase behaviour. The compounds comprising mesogenic groups do not necessarily have to exhibit an LC phase themselves. It is also possible that they show LC phase behaviour only in mixtures with other compounds. For the sake of simplicity, the term "liquid crystal" is used hereinafter for both mesogenic and LC materials.

However, due to the unfavourably high driving voltage required, to the relatively narrow phase range of the chiral nematic materials and to their irreversible switching properties, materials from prior art are not compatible for the use with current LCD driving schemes.

For displays of the USH and ULH mode, new liquid crystalline media with improved properties are required. Especially the birefringence ($\Delta n$) should be optimized for the optical mode. The birefringence $\Delta n$ herein is defined in equation (5)

$$\Delta n = n_e - n_o \qquad (5)$$

wherein ne is the extraordinary refractive index and $n_o$ is the ordinary refractive index, and the average refractive index $n_{av.}$ is given by the following equation (6).

$$n_{av.} = [(2\, n_o^2 + n_e^2)/3]^{1/2} \qquad (6)$$

The extraordinary refractive index $n_e$ and the ordinary refractive index $n_o$ can be measured using an Abbe refractometer. $\Delta n$ can then be calculated from equation (5).

Furthermore, for displays utilizing the USH or ULH mode the optical retardation d*$\Delta n$ (effective) of the liquid crystal media should preferably be such that the equation (7)

$$\sin^2(\pi \cdot d \cdot \Delta n/\lambda) = 1 \qquad (7)$$

wherein

- d is the cell gap and
- $\lambda$ is the wavelength of light

is satisfied. The allowance of deviation for the right hand side of equation (7) is +/- 3 %.

The wavelength of light generally referred to herein is 550 nm, unless explicitly specified otherwise.

The cell gap of the cells preferably is in the range from 1 $\mu$m to 20 $\mu$m, in particular within the range from 2.0 $\mu$m to 10 $\mu$m.

For the ULH/USH mode, the dielectric anisotropy ($\Delta\varepsilon$) should be as small as possible, to prevent unwinding of the helix upon application of the addressing voltage. Preferably $\Delta\varepsilon$ should be slightly higher than 0 and very preferably be 0.1 or more, but preferably 10 or less, more preferably 7 or less and most preferably 5 or less. Herein, the term "dielectrically positive" is used for compounds or components with $\Delta\varepsilon > 3.0$, "dielectrically neutral" with $-1.5 \leq \Delta\varepsilon \leq 3.0$ and "dielectrically negative" with $\Delta\varepsilon < -1.5$. $\Delta\varepsilon$ is determined at a frequency of 1 kHz and at 20 °C. The dielectric anisotropy of the respective compound is determined from the results of a solution of 10 % of the respective individual compound in a nematic host mixture. In case the solubility of the respective compound in the host medium is less than 10 % its concentration is reduced by a factor of 2 until the resultant medium is stable enough at least to allow the determination of its properties. Preferably the concentration is kept at least at 5 %, however, in order to keep the significance of the results a high as possible. The capacitance of the test mixtures are determined both in a cell with homeotropic and with homogeneous alignment. The cell gap of both types of cells is approximately 20 $\mu$m. The voltage applied is a rectangular wave with a frequency of 1 kHz and a root mean square value typically of 0.5 V to 1.0 V, however, it is always selected to be below the capacitive threshold of the respective test mixture.

$\Delta\varepsilon$ is defined as $(\varepsilon\| - \varepsilon_\perp)$, whereas $\varepsilon_{av.}$ is $(\varepsilon\| + 2\,\varepsilon_\perp) / 3$. The dielectric permittivity of the compounds is determined from the change of the respective values of a host medium upon addition of the compounds of interest. The values are

extrapolated to a concentration of the compounds of interest of 100 %. A typical host mixture is disclosed in H.J. Coles et al., J. Appl. Phys. 2006, 99, 034104 and has the composition given in the table.

Table 1: Host mixture composition

| Compound | Concentration |
|---|---|
| F-PGI-ZI-9-Z-GP-F | 25 % |
| F-PGI-ZI-11-Z-GP-F | 25 % |
| F-PGI-O-5-O-PP-N | 9.5 % |
| F-PGI-O-7-O-PP-N | 39 % |
| CD-1 | 1.5 % |

Besides the above mentioned parameters, the media have to exhibit a suitably wide range of the nematic phase, a rather small rotational viscosity and an at least moderately high specific resistivity.

Similar liquid crystal compositions with short cholesteric pitch for flexoelectric devices are known from EP 0 971 016, GB 2 356 629 and Coles, H.J., Musgrave, B., Coles, M.J., and Willmott, J., J. Mater. Chem., 11, p. 2709-2716 (2001). EP 0 971 016 reports on mesogenic estradiols, which, as such, have a high flexoelectric coefficient. GB 2 356 629 discloses broad generic formulae of bimesogenic compounds and suggests the use of these bimesogenic compounds in flexoelectric devices. The flexoelectric effect herein has been investigated in pure cholesteric liquid crystal compounds and in mixtures of homologous compounds only so far. Most of these compounds are used in binary mixtures consisting of a chiral additive and a nematic liquid crystal material being either a simple, conventional monomesogenic material or a bimesogenic one. These materials do have several drawbacks for practical applications, like insufficiently wide temperature ranges of the chiral nematic - or cholesteric phase, too small flexoelectric ratios, small angles of rotation.

Non-symmetrically linked bimesogenic compounds are proposed e.g. in WO 2014/005672 (A).

EP 0 233 688 discloses bis(phenylethanolamines) and bis(phenoxypropanol-amines) useful as beta-agonists.

Macro Rings. 11. Polynuclear Paracyclophanes' H. Steinberg, Donald J Cram, JACS, (1952), 74, p. 5388-91 discloses some compounds.

CN 102617304 discloses difluorphenyl alkoxy ethers like

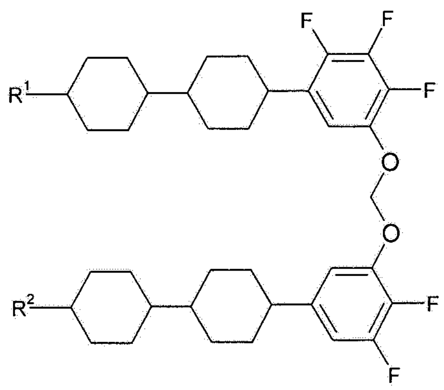

comprising the following moiety

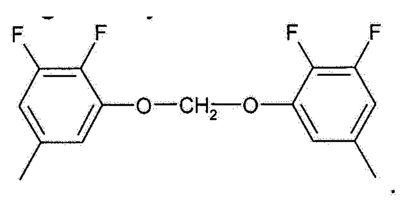

Molecular Crystals and Liquid Crystals (1984). 102 (8-9), pp. 223-233:

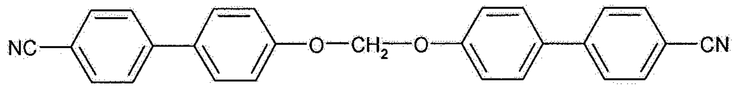

Tetrahedron 27, 36, pp. 4331 to 4334 discloses compounds of formula:

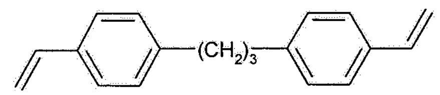

and

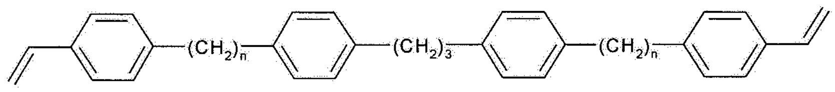

with n selected from 0, 1, 2 and 3 as educts for photocycloaddition of styrene derivatives to cyclophanes.
JP 2006-206464 (A) discloses compounds of the formula

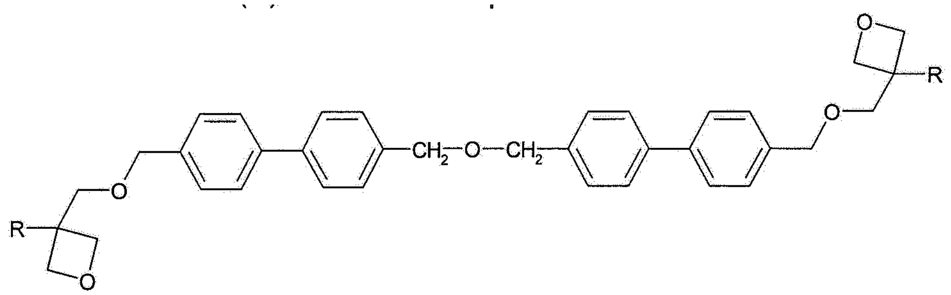

as monomers for cationic polymerization.
In Mahajan R et al: "New mesogens with a fluorosubstituted bent core", Liquid Crystals, Taylor & Francis, vol. 31, no. 2, 1 February 2004 (2004-02-01), pages 161-167,
bimesogens with a spacer having a total length of one atom and with imine or azo groups have have been synthesized and their mesomorphic properties evaluated.
In WO 2006/061094 A1 bimesogenic compounds with a spacer having a total length of one atom are proposed for the use in liquid crystal media with a Blue Phase.
One aim of the invention is to provide improved flexoelectric devices that exhibit high switching angles and fast response times. Another aim is to provide liquid crystal materials with advantageous properties, in particular for use in flexoelectric displays that enable good uniform alignment over the entire area of the display cell without the use of a mechanical shearing process, good contrast, high switching angles and fast response times also at low temperatures. The liquid crystal materials should exhibit low melting points, broad chiral nematic phase ranges, short temperature independent pitch lengths and high flexoelectric coefficients. Other aims of the present invention are immediately evident to the person skilled in the art from the following detailed description.
Using one terminal alkyl group and keeping the other as a dipole inducing group shows a significant effect in improving the alignment of the mixture and maintaining the good flexoelectric properties of the molecule at the same time. Using materials which improve the homeotropic alignment quality of a mixture have shown an improved alignment when placed in the chiral Uniform Lying Helix orientation.
Flexoelectric bimesogenic materials should include a lateral dipole to have the greatest electrooptic flexoelectric response, based on the most widely accepted theories of flexoelectricity. Materials having terminal alkyl chains lose the balanced high polarities, which help provide a dipole across the length of the molecule. Rather, the materials now have a terminal dipole, materials of this terminal alkyl type are also shown to improve the alignment of the liquid crystal system in which they are used.
The inventors have found out that the above aims can be surprisingly achieved by providing bimesogenic compounds according to the present invention. These compounds, when used in chiral nematic liquid crystal mixtures, lead to low melting points, broad chiral nematic phases. In particular, they exhibit relatively high values of the elastic constant $k_{11}$, low values of the bend elastic constant $k_{33}$ and the flexoelectric coefficient.
Thus, the present invention relates to a liquid crystalline medium comprising one or more bimesogenic compounds of formula I and one or more compounds of formula III

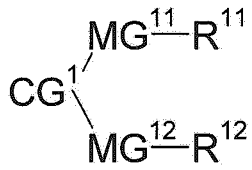

I

$R^{31}$-$MG^{31}$-$X^{31}$-$Sp^3$-$X^{32}$-$MG^{32}$-$R^{32}$ III

wherein

$R^{11}$ and $R^{12}$ are each independently F, Cl, CN, $OCF_3$, or $CF_3$,

$MG^{11}$ and $MG^{12}$ are each independently a mesogenic group,

at least one of

$MG^{11}$ and $MG^{12}$ comprises one, two or more 5-atomic and/or 6-atomic rings, in case of comprising two or more 5- and/or 6-atomic rings at least two of these may be linked by a 2-atomic linking group, preferably selected from the group of linking groups -CO-O-, -O-CO-, $-CH_2$-O-, -O-$CH_2$-, $-CF_2$-O- and -O-$CF_2$-,

preferably $MG^{11}$ and $MG^{12}$ independently of each other comprise 1, 2 or three rings,

preferably $MG^{11}$ and $MG^{12}$ are different from each other, more preferably they comprise a number of rings which is different from one another;

$CG^1$ is a central atom or a central group, having a total length of one atom, preferably selected from $-CH_2$-, -CHF-, $-CF_2$-, -O-, -S-, -(C=O)-, -CH(OR)-, -CH(R)-, -C(R)(R')-, $-SO_2$-, -CH($CF_3$)-, -C(=$CH_2$)-, -NH-, -N(R)-and -S(R)(R')-, more preferably selected from $-CH_2$-, -O-, -(C=O)- and $-SO_2$-, most preferably selected from $-CH_2$- and -O-, and

R and R' are independently of each other an alkyl group having 1 to 5 carbon atoms, wherein one or two non-terminal and non-adjacent $-CH_2$- groups may be replaced by O.

$R^{31}$ and $R^{32}$ are each independently H, F, Cl, CN, NCS or a straight-chain or branched alkyl group with 1 to 25 C atoms which may be unsubstituted, mono- or polysubstituted by halogen or CN, it being also possible for one or more non-adjacent $CH_2$ groups to be replaced, in each case independently from one another, by -O-, -S-, -NH-, -N($CH_3$)-, -CO-, -COO-, -OCO-, -O-CO-O-, -S-CO-, -CO-S-, -CH=CH-, -CH=CF-, -CF=CF- or -C≡C- in such a manner that oxygen atoms are not linked directly to one another,

$MG^{31}$ and $MG^{32}$ are each independently a mesogenic group,

$Sp^3$ is a spacer group comprising 5 to 40 C atoms, wherein one or more non-adjacent $CH_2$ groups may also be replaced by -O-, -S-, -NH-, -N($CH_3$)-, -CO-, -O-CO-, -S-CO-, -O-COO-, -CO-S-, -CO-O-, -CH(halogen)-, -CH(CN)-, -CH=CH- or -C≡C-, and

$X^{31}$ and $X^{32}$ are each independently -O-, -S-, -CO-, -COO-, -OCO-, -O-CO-O-, -CO-NH-, -NH-CO-, $-CH_2CH_2$-, $-OCH_2$-, $-CH_2O$-, $-SCH_2$-, $-CH_2S$-, -CH=CH-, -CH=CH-COO-, -OCO-CH=CH-, -C≡C- or a single bond, and

with the condition that compounds of formula I are excluded from compounds of formula III.

preferably $MG^{11}$ and $MG^{12}$ are different from each other, more preferably they comprise a number of rings which is different from one another,

$CG^1$ is a central atom or a central group, having a total length of one atom, preferably selected from $-CH_2-$, -CHF-, $-CF_2-$, -O-, -S-, -(C=O)-, -CH(OR)-, -CH(R)-, -C(R)(R')-, $-SO_2-$, $-CF_2-$, $-CH(CF_3)-$, $-C(=CH_2)-$, -NH-,-N(R)- and -S(R)(R')-, more preferably selected from $-CH_2-$, -O-, -(C=O)- and $-SO_2-$, most preferably selected from $-CH_2-$ and -O-, and

R and R' are independently of each other an alkyl group having 1 to 5 carbon atoms, wherein one or two non-terminal and non-adjacent $-CH_2-$ groups may be replaced by O.

In a first preferred embodiment

$R^{11}$ is H, F, Cl, CN, CF3, OCF3 or a straight-chain or branched alkyl group with 1 to 25 C atoms, and

$R^{12}$ is H, F, Cl, CN, CF3, OCF3 or a straight-chain or branched alkyl group with 1 to 25 C atoms which may be unsubstituted, mono- or polysubstituted by halogen or CN, and/or

$MG^{11}$ and/or $MG^{12}$ is a mesogenic group, which comprises one or more 6-atomic rings, optionally substituted by F, Cl, CN, $OCH_3$, $OCF_3$ at least two of these may be linked by a 2-atomic linking group, preferably selected from the group of linking groups -CO-O-, -O-CO-, $-CH_2-O-$, $-O-CH_2-$, $-CF_2-O-$ and $-O-CF_2-$, -CH=CH-, -C≡C-, $-C(O)-CH_2-$, $-CH_2-CHF_2-$, or $-CF_2-CF_2-$, preferably selected from -CO-O-, -O-CO-, $-CH_2-O-$, $-O-CH_2-$, $-CF_2-O-$ and $-O-CF_2-$.

In a preferred embodiment, which may be different from or identical to any of the preferred embodiments, at least one of

$MG^{11}$ and $MG^{12}$ comprises two or more 5- or 6-atomic rings, at least two of which are linked by a 2-atomic linking group, preferably selected from the group of linking groups -CO-O-, -O-CO-, $-CH_2-O-$, $-O-CH_2-$, $-CF_2-O-$ and $-O-CF_2-$.

Further preferred compounds of formula I are compounds in which

$MG^{11}$ and $MG^{12}$ are independently from one another a group of (partial) formula II

$$-A^{11}-(Z^{11}-A^{12})_k- \qquad \text{II}$$

wherein

$Z^{11}$ are, independently of each other in each occurrence, a single bond, -COO-, -OCO-, -O-CO-O-, $-OCH_2-$, $-CH_2O-$, -OCF2-, $-CF_2O-$, $-CH_2CH_2-$, $-(CH_2)_4-$, $-CF_2CF_2-$, -CH=CH-, -CF=CF-, -CH=CH-COO-, -OCO-CH=CH- or -C≡C-, optionally substituted with one or more of F, S and/or Si, preferably a single bond,

$A^{11}$ and $A^{12}$ are each independently in each occurrence 1,4-phenylene, wherein in addition one or more CH groups may be replaced by N, trans-1,4-cyclo-hexylene in which, in addition, one or two non-adjacent $CH_2$ groups may be replaced by O and/or S, 1,4-cyclohexenylene, 1,4-bicyclo-(2,2,2)-octylene, piperidine-1,4-diyl, naphthalene-2,6-diyl, decahydro-naphthalene-2,6-diyl, 1,2,3,4-tetrahydro-naphthalene-2,6-diyl, cyclobutane-1,3-diyl, spiro[3.3] heptane-2,6-diyl or dispiro[3.1.3.1] decane-2,8-diyl, it being possible for all these groups to be unsubstituted, mono-, di-, tri- or tetrasubstituted with F, Cl, CN or alkyl, alkoxy, alkylcarbonyl or alkoxycarbonyl groups with 1 to 7 C atoms, wherein one or more H atoms may be substituted by F or Cl, preferably F, Cl, $CH_3$ or $CF_3$, and in all of these groups one or more non-adjacent $-CH_2-$ groups may be replaced by O or S, preferably by O and one or more non-adjacent -CH= groups may be replaced by N,

alternatively one or more of $A^{11}$ and $A^{12}$ are each independently in each occurrence a comprise a 5-atomic ring, and are preferably selected from thiophene-2,5-diyl, furane-2,5-diyl, thiazole-diyl, thiadiazole-diyl, it being possible for all these groups to be unsubstituted, mono-, di-, tri- or tetrasubstituted with F, Cl, CN or alkyl, alkoxy, alkylcarbonyl or alkoxycarbonyl groups with 1 to 7 C atoms, wherein one or more H atoms may be substituted by F or Cl, preferably F, Cl, $CH_3$ or $CF_3$, and the other,

k is 0, 1, 2, 3 or 4, preferably 1, 2 or 3 and, most preferably 1 or 2.

A smaller group of preferred mesogenic groups of formula II comprising only 6-membered rings is listed below. For reasons of simplicity, Phe in these groups is 1,4-phenylene, PheL is a 1,4-phenylene group which is substituted by 1 to 4 groups L, with L being preferably F, Cl, CN, OH, $NO_2$ or an optionally fluorinated alkyl, alkoxy or alkanoyl group with 1 to 7 C atoms, very preferably F, Cl, CN, OH, $NO_2$, $CH_3$, $C_2H_5$, $OCH_3$, $OC_2H_5$, $COCH_3$, $COC_2H_5$, $COOCH_3$, $COOC_2H_5$, $CF_3$, $OCF_3$, $OCHF_2$, $OC_2F_5$, in particular F, Cl, CN, $CH_3$, $C_2H_5$, $OCH_3$, $COCH_3$ and $OCF_3$, most preferably F, Cl, $CH_3$, $OCH_3$ and $COCH_3$ and Cyc is 1,4-cyclohexylene. This list comprises the sub-formulae shown below as well as their mirror images

| | |
|---|---|
| -Phe-Z-Phe- | II-1 |
| -Phe-Z-Cyc- | II-2 |
| -Cyc-Z-Cyc- | II-3 |
| -Phe-Z-PheL- | II-4 |
| -PheL-Z-Phe- | II-5 |
| -PheL-Z-Cyc- | II-6 |
| -PheL-Z-PheL- | II-7 |
| -Phe-Z-Phe-Z-Phe- | II-8 |
| -Phe-Z-Phe-Z-Cyc- | II-9 |
| -Phe-Z-Cyc-Z-Phe- | II-10 |
| -Cyc-Z-Phe-Z-Cyc- | II-11 |
| -Phe-Z-Cyc-Z-Cyc- | II-12 |
| -Cyc-Z-Cyc-Z-Cyc- | II-13 |
| -Phe-Z-Phe-Z-PheL- | II-14 |
| -Phe-Z-PheL-Z-Phe- | II-15 |
| -PheL-Z-Phe-Z-Phe- | II-16 |
| -PheL-Z-Phe-Z-PheL- | II-17 |
| -PheL-Z-PheL-Z-Phe- | II-18 |
| -PheL-Z-PheL-Z-PheL- | II-19 |
| -Phe-Z-PheL-Z-Cyc- | II-29 |
| -Phe-Z-Cyc-Z-PheL- | II-21 |
| -Cyc-Z-Phe-Z-PheL- | II-22 |
| -PheL-Z-Cyc-Z-PheL- | II-23 |
| -PheL-Z-PheL-Z-Cyc- | II-24 |
| -PheL-Z-Cyc-Z-Cyc- | II-25 |
| -Cyc-Z-PheL-Z-Cyc- | II-26 |

wherein

Cyc is 1,4-cyclohexlene, preferably trans-1,4-cyclohexlene,

Phe is 1,4-phenylene,

PheL is 1,4-phenylene, which is substituted by one, two or three fluorine atoms, by one or two Cl atoms or by one Cl atom and one F atom, and

Z has one of the meanings of $Z^{11}$ as given under partial formula II, at least one is preferably selected from -COO-, -OCO-, -O-CO-O-, $-OCH_2-$, $-CH_2O-$, $-OCF_2-$ or $-CF_2O-$.

Particularly preferred are the sub-formulae II-1, II-4, II-5, II-7, II-8, II-14, II-15, II-16, II-17, II-18 and II-19.

In these preferred groups Z in each case independently has one of the meanings of $Z^{11}$ as given under formula

I. Preferably one of Z is -COO-, -OCO-, $-CH_2-O-$, $-O-CH_2-$, $-CF_2-O-$ or $-O-CF_2-$, more preferably -COO-, $-O-CH_2-$ or $-CF_2-O-$, and the others preferably are a single bond.

Very preferably at least one of the mesogenic groups $MG^{11}$ and $MG^{12}$ is, and preferably both of them are each and independently, selected from the following formulae IIa to IIn and their mirror images

| | |
|---|---|
| 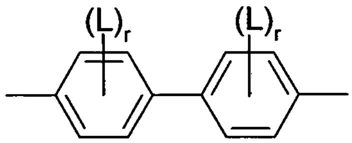 | IIa |
| 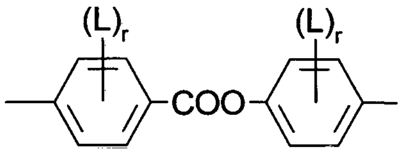 | IIb |
| 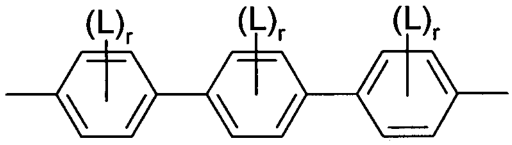 | IIc |
| 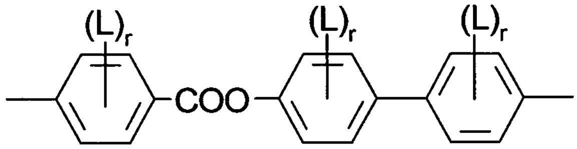 | IId |
| 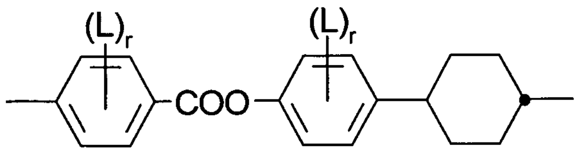 | IIe |
| 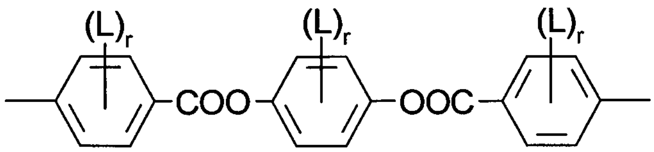 | IIf |
| 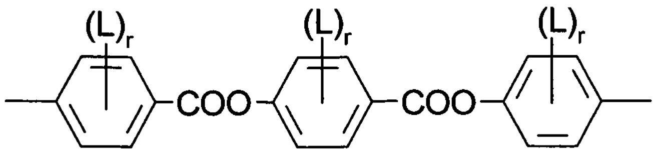 | IIg |
| 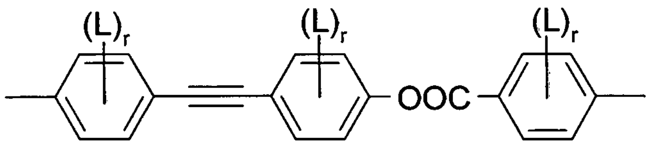 | IIh |
| 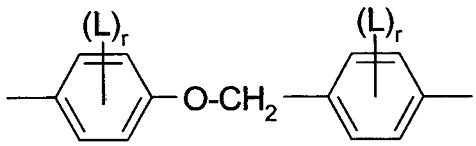 | II i |

(continued)

| | |
|---|---|
| 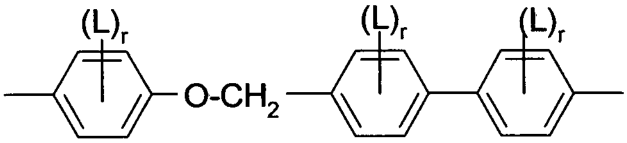 | II j |
| 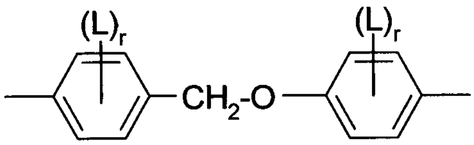 | IIk |
| 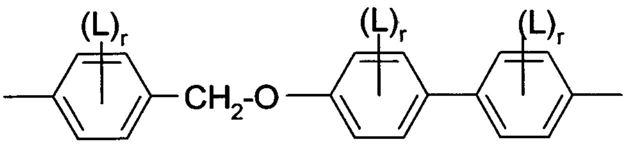 | II l |
| 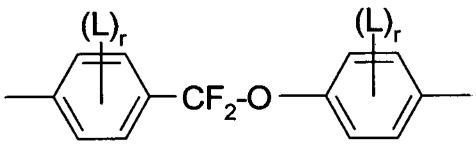 | IIm |
| 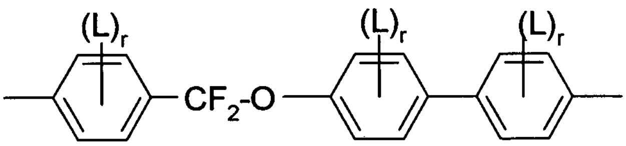 | IIn |
| 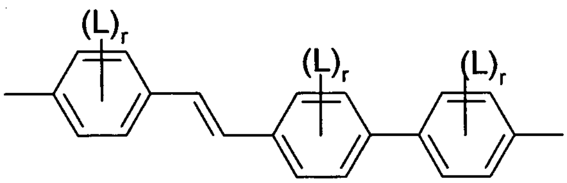 | IIo |

wherein

L is in each occurrence independently of each other F or Cl, preferably F and

r is in each occurrence independently of each other 0, 1, 2 or 3, preferably 0, 1 or 2.

The group

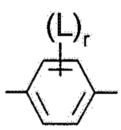

in these preferred formulae is very preferably denoting

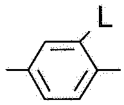
,
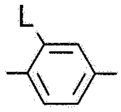
or
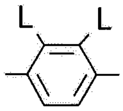
,

furthermore

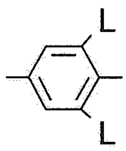
,

L is in each occurrence independently of each other F or Cl, F.

"Terminal" $CH_2$ groups are those directly bonded to the mesogenic groups. Accordingly, "non-terminal" $CH_2$ groups are not directly bonded to the mesogenic groups $MG^{11}$ and $MG^{12}$.

Oxaalkyl, i.e. where one $CH_2$ group is replaced by -O-, is preferably straight-chain 2-oxapropyl (=methoxymethyl), 2- (=ethoxymethyl) or 3-oxabutyl (=2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Preferred are compounds of formula I wherein the mesogenic groups $R^{11}$-$MG^{11}$ and $R^{12}$-$MG^{12}$ are different from each other. In another embodiment compounds of formula I wherein $R^{11}$-$MG^{11}$- and $R^{12}$-$MG^{12}$- in formula I are identical to each other.

Preferred compounds of formula I are selected from the group of compounds of formulae I-1A to I-1E, I-2A to I-2E and I-3A to I-3E,

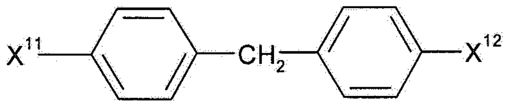
I-1A

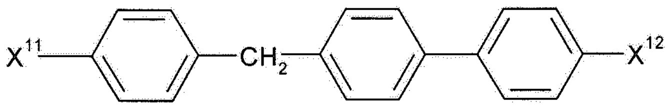
I-1B

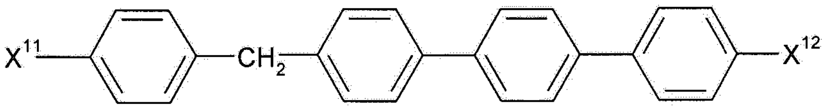
I-1C

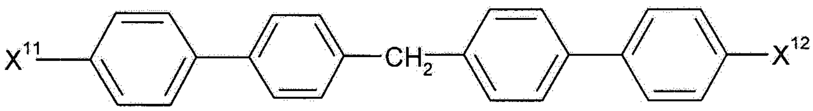
I-1D

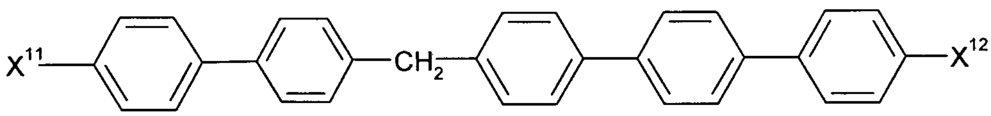
I-1E

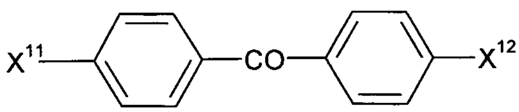
I-2A

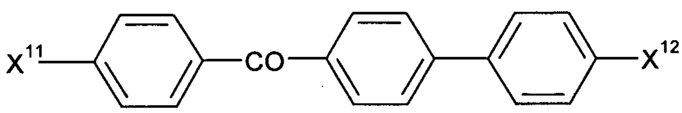
I-2B

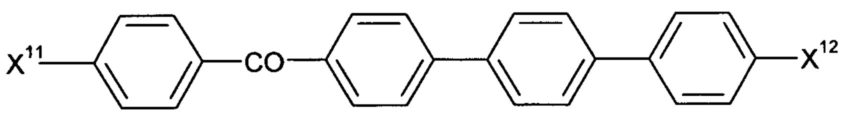
I-2C

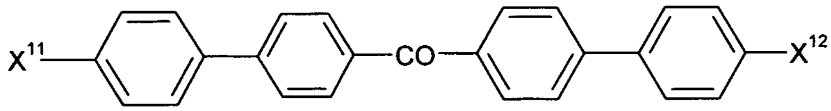
I-2D

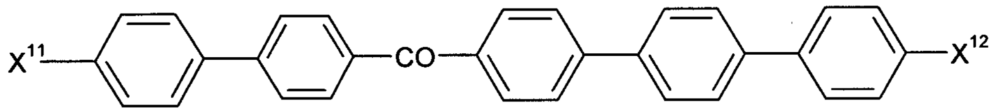
I-2E

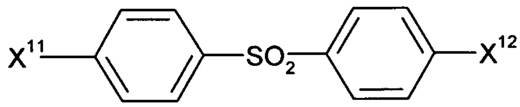
I-3A

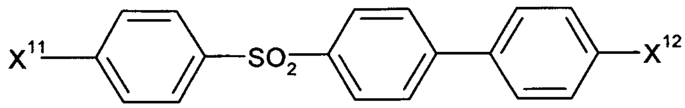
I-3B

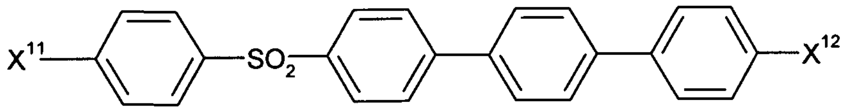
I-3C

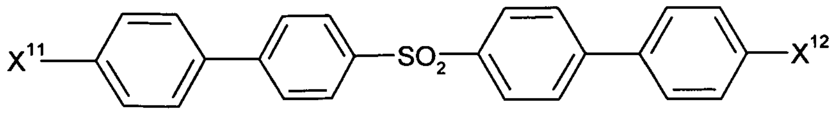
I-3D

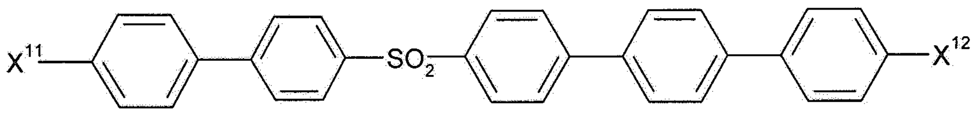
I-3E

wherein the $X^{11}$ and $X^{12}$ have the respective meanings given for $R^{11}$ and $R^{12}$ under formula I above, and wherein one or more of the 1,4-phenylene moieties may optionally substituted by one, two or three, preferably by one or two, most preferably by one substituent L selected from the group of substituents F, Cl, CN, methyl or ethyl, preferably selected from F and Cl and, most preferably by F.

$R^{11}$ and $R^{12}$ are independently from each other as defined above, including the preferred meanings of these groups, preferably $R^{11}$ is F or CN, preferably $R^{12}$ is $OCF_3$, $CF_3$, F or CN, more preferably F or CN and most preferably CN and wherein L is in each occurrence independently of each other F, Cl or preferably F or Cl, most preferably F.

Particularly preferred compounds are selected from the group of formulae given above, which bear 0, 2 or 4 F atoms in lateral positions (i.e. as L).

In a preferred embodiment of the present invention $R^{11}$ is $OCF_3$ and $R^{12}$ is $OCF_3$, F or CN, preferably $OCF_3$ or CN and most preferably CN.

The compounds of formula I can be synthesized according to or in analogy to methods which are known per se and which are described in standard works of organic chemistry such as, for example, Houben-Weyl, "Methoden der organischen Chemie", Thieme-Verlag, Stuttgart. A preferred method of preparation can be taken from the following synthesis schemes.

The compounds of formula I are preferably accessible according to known reactions.

Compounds of formula I, when added to a nematic liquid crystalline mixture, produce a phase below the nematic. In this context, a first indication of the influence of bimesogenic compounds on nematic liquid crystal mixtures was reported by Barnes, P.J., Douglas, A.G., Heeks, S.K., Luckhurst, G.R., Liquid Crystals, 1993, Vol.13, No.4, 603- 613. This reference exemplifies highly polar alkyl spacered dimers and perceives a phase below the nematic, concluding it is a type of smectic.

A photo evidence of an existing mesophase below the nematic phase was published by Henderson, P.A., Niemeyer, O., Imrie, C.T. in Liquid Crystals, 2001, Vol. 28, No.3, 463-472, which was not further investigated.

In Liquid Crystals, 2005, Vol. 32, No. 11-12, 1499-1513 Henderson, P.A., Seddon, J.M. and Imrie, C.T. reported that the new phase below the nematic belonged in some special examples to a smectic C phase. A additional nematic phase below the first nematic was reported by Panov, V.P., Ngaraj, M., Vij, J.K., Panarin, Y.P., Kohlmeier, A., Tamba, M.G., Lewis, R.A. and Mehl, G.H. in Phys.Rev.Lett. 2010, 105, 1678011 - 1678014.

In this context, liquid crystal mixtures comprising the bimesogenic compounds of formula I may show also a novel mesophase that is being assigned as a second nematic phase. This mesophase exists at a lower temperature than the original nematic liquid crystalline phase and has been observed in the unique mixture concepts presented by this invention.

Accordingly, the bimesogenic compounds of formula I allow the second nematic phase to be induced in nematic mixtures that do not have this phase normally. Furthermore, varying the amounts of compounds of formula I allow the phase behaviour of the second nematic to be tailored to the required temperature.

Some preferred embodiments of the mixtures according to the invention are indicated below.

Preferred are compounds of formula I wherein the mesogenic groups $MG^{11}$ and $MG^{12}$ at each occurrence independently from each other comprise one, two or three six-membered rings, preferably two or three six-membered rings.

Particularly preferred are the partial formulae II-1, II-4, II-6, II-7, II-13, II-14, II-15, II-16, II-17 and I-18.

Preferably $R^{11}$ and $R^{12}$ in formula I are selected of F, CN and $OCF_3$.

Preferred are compounds of formula I wherein $R^{11}$-$MG^{11}$ and $R^{12}$-$MG^{12}$- in formula I are identical.

The media according to the invention preferably comprise one, two, three, four or more, preferably one, two or three, compounds of the formula I.

The amount of compounds of formula I in the liquid crystalline medium is preferably from 1 to 50 %, in particular from 5 to 40 %, very preferably 10 to 30 % by weight of the total mixture.

In formula III, the mesogenic groups $MG^{31}$ and $MG^{32}$ are preferably selected of formula II above.

Especially preferred are compounds of formula III wherein $R^{31}$-$MG^{31}$-$X^{31}$- and $R^{32}$-$MG^{32}$-$X^{32}$- are identical.

Another preferred embodiment of the present invention relates to compounds of formula III wherein $R^{31}$-$MG^{31}$-$X^{31}$- and $R^{32}$-$MG^{32}$-$X^{32}$- are different.

Especially preferred are compounds of formula III wherein the mesogenic groups $MG^{31}$ and $MG^{32}$ comprise one, two or three six-membered rings very preferably are the mesogenic groups selected from formula II as listed below.

For $MG^{31}$ and $MG^{32}$ in formula III are particularly preferred are the subformulae II-1, II-4, II-6, II-7, II-13, II-14, II-15, II-16, II-17 and II-18. In these preferred groups Z in each case independently has one of the meanings of $Z^1$ as given in formula II. Preferably Z is -COO-, -OCO-, $-CH_2CH_2-$, -C≡C- or a single bond.

Very preferably the mesogenic groups $MG^{31}$ and $MG^{32}$ are selected from the formulae IIa to IIo and their mirror images.

In case of compounds with a non-polar group, $R^{31}$ and $R^{32}$ are preferably alkyl with up to 15 C atoms or alkoxy with 2 to 15 C atoms.

If $R^{31}$ or $R^{32}$ is an alkyl or alkoxy radical, i.e. where the terminal $CH_2$ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one $CH_2$ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

In case of a compounds with a terminal polar group, $R^{31}$ and $R^{32}$ are selected from CN, $NO_2$, halogen, $OCH_3$, OCN, SCN, $COR^x$, $COOR^x$ or a mono- oligo- or polyfluorinated alkyl or alkoxy group with 1 to 4 C atoms. $R^x$ is optionally fluorinated alkyl with 1 to 4, preferably 1 to 3 C atoms. Halogen is preferably F or Cl.

Especially preferably $R^{31}$ and $R^{32}$ in formula III are selected of F, Cl, CN, $NO_2$, $OCH_3$, $COCH_3$, $COC_2H_5$, $COOCH_3$, $COOC_2H_5$, $CF_3$, $C_2F_5$, $OCF_3$, $OCHF_2$, and $OC_2F_5$, in particular of F, Cl, CN, $OCH_3$ and $OCF_3$.

As for the spacer group $Sp^3$ in formula III all groups can be used that are known for this purpose to the skilled in the art. The spacer group Sp is preferably a linear or branched alkylene group having 5 to 40 C atoms, in particular 5 to 25 C atoms, very preferably 5 to 15 C atoms, in which, in addition, one or more non-adjacent $CH_2$ groups may be replaced by -O-, -S-, -NH-, $-N(CH_3)-$, -CO-, -O-CO-, -S-CO-, -O-COO-, -CO-S-, -CO-O-, -CH(halogen)-, -CH(CN)-, -CH=CH- or -C≡C-.

Typical spacer groups are for example $-(CH_2)_o-$, $-(CH_2CH_2O)_p-CH_2CH_2-$, $-CH_2CH_2-S-CH_2CH_2-$ or $-CH_2CH_2-NH-CH_2CH_2-$, with o being an integer from 5 to 40, in particular from 5 to 25, very preferably from 5 to 15, and p being an integer from 1 to 8, in particular 1, 2, 3 or 4.

Preferred spacer groups are pentylene, hexylene, heptylene, octylene, nonylene, decylene, undecylene, dodecylene, octadecylene, diethyleneoxyethylene, dimethyleneoxybutylene, pentenylene, heptenylene, nonenylene and undecenylene, for example.

Especially preferred are inventive compounds of formula III wherein $Sp^3$ is denoting alkylene with 5 to 15 C atoms. Straight-chain alkylene groups are especially preferred.

In another preferred embodiment of the invention the chiral compounds of formula III comprise at least one spacer group $Sp^1$ that is a chiral group of the formula IV.

$X^{31}$ and $X^{32}$ in formula III denote preferably -O-, -CO-, -COO-, -OCO-, -O-CO-O- or a single bond. Particularly preferred are the following compounds selected from formulae III-1 to III-4:

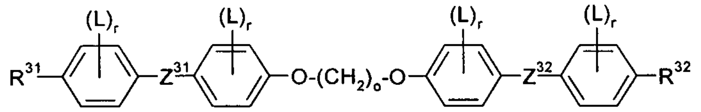

III-1

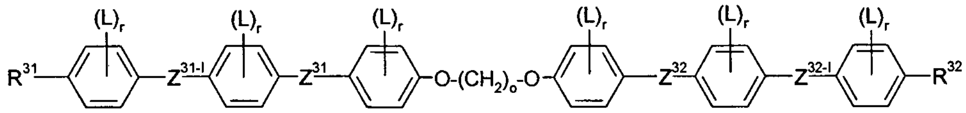

III-2

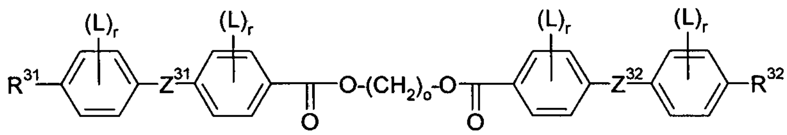

III-3

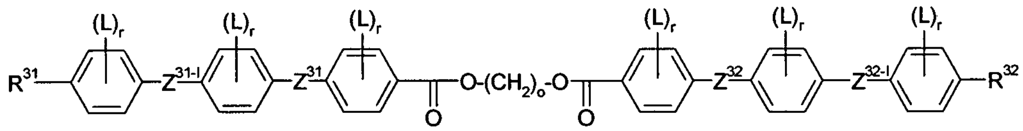

III-4

wherein $R^{31}$, $R^{32}$ have the meaning given under formula III, $Z^{31}$ and $Z^{31-I}$ are defined as $Z^{31}$ and $Z^{32}$ and $Z^{32-I}$ are respectively the reverse groups of $Z^{31}$ and $Z^{32-I}$ in formula III and o and r are independently at each occurrence as defined above, including the preferred meanings of these groups and wherein L is in each occurrence independently of each other preferably F, Cl, CN, OH, $NO_2$ or an optionally fluorinated alkyl, alkoxy or alkanoyl group with 1 to 7 C atoms, very preferably F, Cl, CN, OH, $NO_2$, $CH_3$, $C_2H_5$, $OCH_3$, $OC_2H_5$, $COCH_3$, $COC_2H_5$, $COOCH_3$, $COOC_2H_5$, $CF_3$, $OCF_3$, $OCHF_2$, $OC_2F_5$, in particular F, Cl, CN, $CH_3$, $C_2H_5$, $OCH_3$, $COCH_3$ and $OCF_3$, most preferably F, Cl, $CH_3$, $OCH_3$ and $COCH_3$ and from which compounds of formula I are excluded.

Particularly preferred mixtures according to the invention comprise one or more compounds of the formulae III-1a to III-1e and III-3a to III-3b.

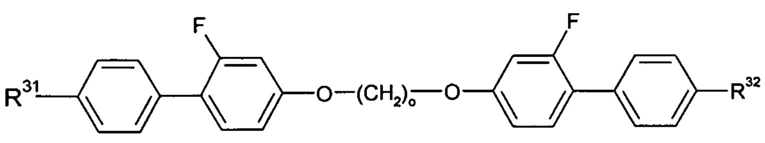

III-1a

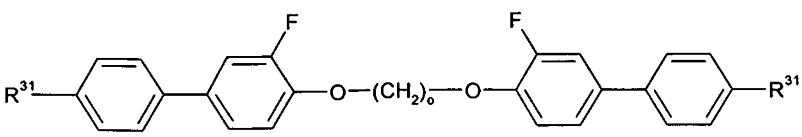

III-1b

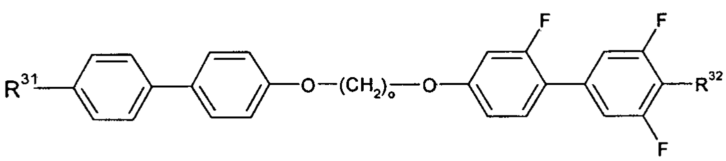

III-1c

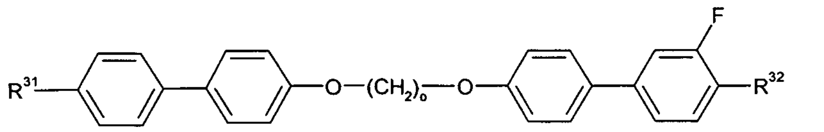

III-1d

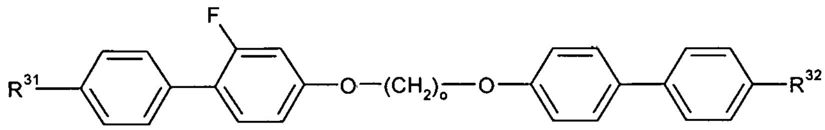

III-1e

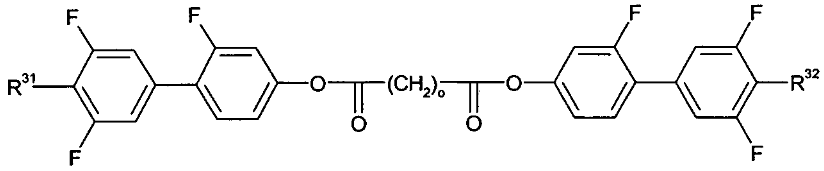

III-3a

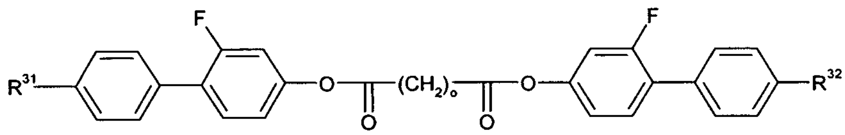

III-3b

wherein the parameters are as defined above.

In a preferred embodiment of the invention the liquid crystalline medium is consisting of 2 to 25, preferably 3 to 15 compounds of formula III.

The amount of compounds of formula III in the liquid crystalline medium is preferably from 10 to 95 %, in particular from 15 to 90 %, very preferably 20 to 85 % by weight of the total mixture.

Preferably, the proportion of compounds of the formulae III-1a and/or III-1b and/or III-1c and/or III-1e and or III-3a and/or III-3b in the medium as a whole is preferably at least 70 % by weight.

Particularly preferred media according to the invention comprise at least one or more chiral dopants which themselves do not necessarily have to show a liquid crystalline phase and give good uniform alignment themselves.

Especially preferred are chiral dopants selected from formula IV

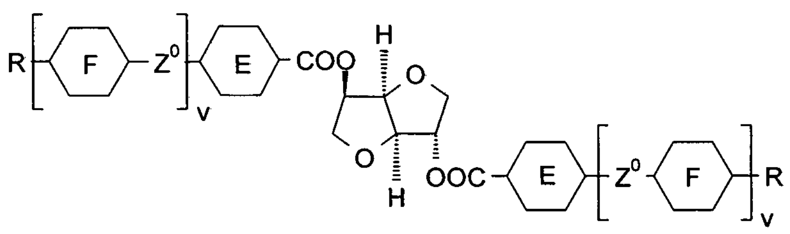

IV

and formula V

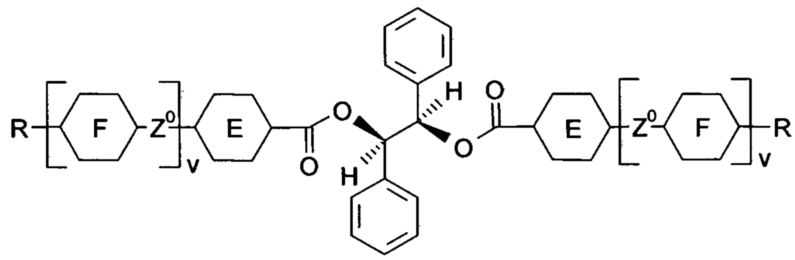

V

including the respective (S,S) enantiomer,

wherein E and F are each independently 1,4-phenylene or trans-1,4-cyclohexylene, v is 0 or 1, $Z^0$ is -COO-, -OCO-, $-CH_2CH_2-$ or a single bond, and R is alkyl, alkoxy or alkanoyl with 1 to 12 C atoms.

The compounds of formula IV and their synthesis are described in WO 98/00428. Especially preferred is the compound CD-1, as shown in table D below. The compounds of formula V and their synthesis are described in GB 2,328,207.

Especially preferred are chiral dopants with a high helical twisting power (HTP), in particular those disclosed in WO 98/00428.

Further typically used chiral dopants are e.g. the commercially available R/S-5011, CD-1, R/S-811 and CB-15 (from Merck KGaA, Darmstadt, Germany).

The above mentioned chiral compounds R/S-5011 and CD-1 and the compounds of formula IV and V exhibit a very high helical twisting power (HTP), and are therefore particularly useful for the purpose of the present invention.

The liquid crystalline medium preferably comprises preferably 1 to 5, in particular 1 to 3, very preferably 1 or 2 chiral dopants, preferably selected from the above formula IV, in particular CD-1, and/or formula V and/or R-5011 or S-5011, very preferably the chiral compound is R-5011, S-5011 or CD-1.

The amount of chiral compounds in the liquid crystalline medium is preferably from 1 to 20 %, in particular from 1 to 15 %, very preferably 1 to 10 % by weight of the total mixture.

Further preferred are liquid crystalline media comprising one or more additives selected from the following formula VI

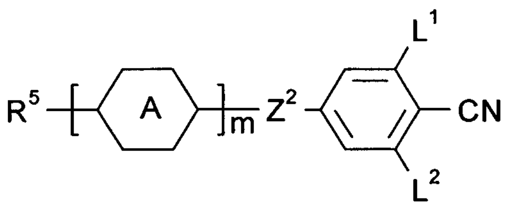

VI

wherein

$R^5$ is alkyl, alkoxy, alkenyl or alkenyloxy with up to 12 C atoms,

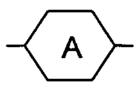

is

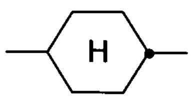 , 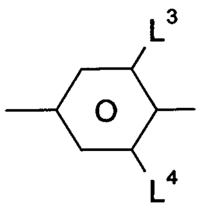 , 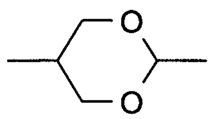 or 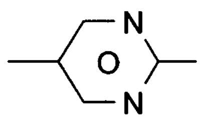 ,

$L^1$ through $L^4$ are each independently H or F,
$Z^2$ is -COO-, $-CH_2CH_2-$ or a single bond,
m is 1 or 2

Particularly preferred compounds of formula VI are selected from the following formulae

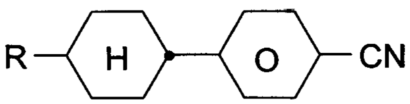

VIa

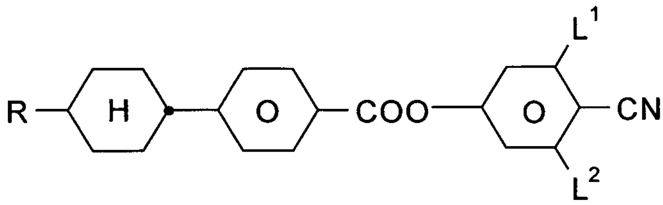

VIb

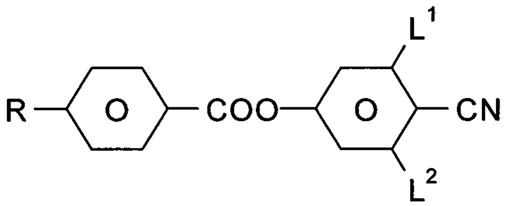

VIc

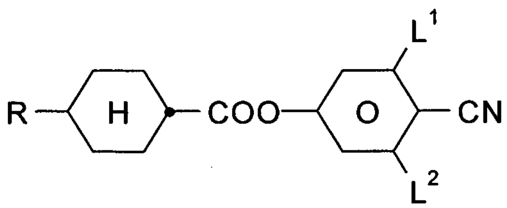

VId

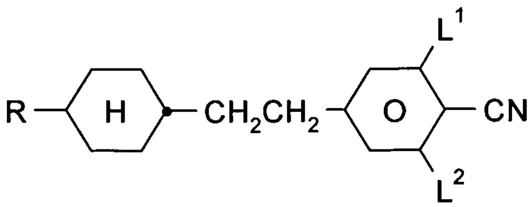

VIe

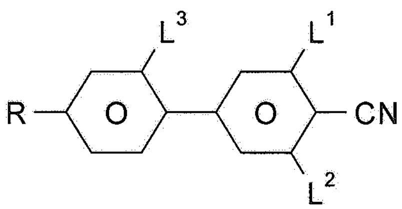

VIf

wherein, R has one of the meanings of $R^5$ above and $L^1$, $L^2$ and $L^3$ have the above meanings.

The liquid crystalline medium preferably comprises 1 to 5, in particular 1 to 3, very preferably 1 or 2, preferably selected from the above formulae Via to VIf, very preferably from formulae VIf.

The amount of suitable additives of formula VI in the liquid crystalline medium is preferably from 1 to 20 %, in particular from 1 to 15 %, very preferably 1 to 10 % by weight of the total mixture.

The liquid crystal media according to the present invention may contain further additives in usual concentrations. The total concentration of these further constituents is in the range of 0.1 % to 10 %, preferably 0.1 % to 6 %, based on the total mixture. The concentrations of the individual compounds used each are preferably in the range of 0.1 % to 3 %. The concentration of these and of similar additives is not taken into consideration for the values and ranges of the concentrations of the liquid crystal components and compounds of the liquid crystal media herein. This also holds for the concentration of the dichroic dyes used in the mixtures, which are not counted when the concentrations of the compounds respectively the components of the host medium are specified. The concentration of the respective additives is always given relative to the final doped mixture.

The liquid crystal media according to the present invention consists of several compounds, preferably of 3 to 30, more preferably of 4 to 20 and most preferably of 4 to 16 compounds. These compounds are mixed in conventional way. As a rule, the required amount of the compound used in the smaller amount is dissolved in the compound used in the greater amount. In case the temperature is above the clearing point of the compound used in the higher concentration, it is particularly easy to observe completion of the process of dissolution. It is, however, also possible to prepare the

media by other conventional ways, e.g. using so called pre-mixtures, which can be e.g. homologous or eutectic mixtures of compounds or using so called multi-bottle-systems, the constituents of which are ready to use mixtures themselves.

Particularly preferred mixture concepts are indicated below: (the acronyms used are explained in Table A).

The mixtures according to the invention preferably comprise

- one or more compounds of formula I in a total concentration in the range from 1 to 50 %, in particular from 5 to 40 %, very preferably 10 to 30 % by weight of the total mixture
  and/or

- one or more compounds of formula III in a total concentration in the range from 10 to 95 %, in particular from 15 to 90 %, very preferably 20 to 85 % by weight of the total mixture, preferably these compounds are selected from formulae III-1a to III-1e and III-3a to III-3b especially preferred they comprise

  - N-PGI-ZI-n-Z-GP-N, preferably N-PGI-ZI-7-Z-GP-N and/or N-PGI-ZI-9-Z-GP-N preferably in concentrations > 5 %, in particular 10-30 %, based on the mixture as a whole,
    and/or

  - F-UIGI-ZI-n-Z-GU-F, preferably F-UIGI-ZI-9-Z-GU-F, preferably in concentrations > 5 %, in particular 10-30 %, based on the mixture as a whole,
    and/or

  - F-PGI-O-n-O-PP-N, preferably F-PGI-O-9-O-PP-, preferably in concentrations of > 1 %, in particular 1-20 %, based on the mixture as a whole,
    and/or

  - N-PP-O-n-O-PG-OT, preferably N-PP-O-7-O-PG-OT, preferably in concentrations of > 5 %, in particular 5-30 %, based on the mixture as a whole,
    and/or

  - N-PP-O-n-O-GU-F, preferably N-PP-O-9-O-GU-F, preferably in concentrations of > 1 %, in particular 1-20 %, based on the mixture as a whole,
    and/or

  - F-PGI-O-n-O-GP-F, preferably F-PGI-O-7-O-GP-F and/or F-PGI-O-9-O-GP-F preferably in concentrations of > 1 %, in particular 1-20 %, based on the mixture as a whole,
    and/or

  - N-GIGIGI-n-GGG-N, in particular N-GIGIGI-9-GGG-N, preferably in concentration > 5 %, in particular 10-30 %, based on the mixture as a whole,
    and/or

  - N-PGI-n-GP-N, preferably N-PGI-9-GP-N, preferably in concentrations > 5 %, in particular 15-50 %, based on the mixture as a whole,
    and/or

- one or more suitable additives of formula VI in a total concentration in the range from 1 to 20 %, in particular from 1 to 15 %, very preferably 1 to 10 % by weight of the total mixture, preferably are these compounds selected from formula VIa to VIf, especially preferred they comprise

  - PP-n-N, preferably in concentrations of > 1 %, in particular 1-20 %, based on the mixture as a whole,

  and/or

- one or more chiral compounds preferably in a total concentration in the range from 1 to 20 %, in particular from 1 to 15 %, very preferably 1 to 10 % by weight of the total mixture, preferably these compounds are selected from formula IV, V, and R-5011 or S-5011, especially preferred they comprise

  - R-5011, S-5011 or CD-1, preferably in a concentration of > 1 %, in particular 1-20 %, based on the mixture as

a whole.

The bimesogenic compounds of formula I and the liquid crystalline media comprising them can be used in liquid crystal displays, such as STN, TN, AMD-TN, temperature compensation, guest-host, phase change or surface stabilized or polymer stabilized cholesteric texture (SSCT, PSCT) displays, in particular in flexoelectric devices, in active and passive optical elements like polarisers, compensators, reflectors, alignment layers, colour filters or holographic elements, in adhesives, synthetic resins with anisotropic mechanical properties, cosmetics, diagnostics, liquid crystal pigments, for decorative and security applications, in nonlinear optics, optical information storage or as chiral dopants.

The compounds of formula I and the mixtures obtainable thereof are particularly useful for flexoelectric liquid crystal display. Thus, another object of the present invention is a flexoelectric display comprising one or more compounds of formula I or comprising a liquid crystal medium comprising one or more compounds of formula I.

The inventive bimesogenic compounds of formula I and the mixtures thereof can be aligned in their cholesteric phase into different states of orientation by methods that are known to the expert, such as surface treatment or electric fields. For example, they can be aligned into the planar (Grandjean) state, into the focal conic state or into the homeotropic state. Inventive compounds of formula I comprising polar groups with a strong dipole moment can further be subjected to flexoelectric switching, and can thus be used in electrooptical switches or liquid crystal displays.

The switching between different states of orientation according to a preferred embodiment of the present invention is exemplarily described below in detail for a sample of an inventive compound of formula I.

According to this preferred embodiment, the sample is placed into a cell comprising two plane-parallel glass plates coated with electrode layers, e.g. ITO layers, and aligned in its cholesteric phase into a planar state wherein the axis of the cholesteric helix is oriented normal to the cell walls. This state is also known as Grandjean state, and the texture of the sample, which is observable e.g. in a polarization microscope, as Grandjean texture. Planar alignment can be achieved e.g. by surface treatment of the cell walls, for example by rubbing and/or coating with an alignment layer such as polyimide.

A Grandjean state with a high quality of alignment and only few defects can further be achieved by heating the sample to the isotropic phase, subsequently cooling to the chiral nematic phase at a temperature close to the chiral nematic-isotropic phase transition, and rubbing the cell.

In the planar state, the sample shows selective reflection of incident light, with the central wavelength of reflection depending on the helical pitch and the mean refractive index of the material.

When an electric field is applied to the electrodes, for example with a frequency from 10 Hz to 1 kHz, and an amplitude of up to 12 $V_{rms}/\mu m$, the sample is being switched into a homeotropic state where the helix is unwound and the molecules are oriented parallel to the field, i.e. normal to the plane of the electrodes. In the homeotropic state, the sample is transmissive when viewed in normal daylight, and appears black when being put between crossed polarisers.

Upon reduction or removal of the electric field in the homeotropic state, the sample adopts a focal conic texture, where the molecules exhibit a helically twisted structure with the helical axis being oriented perpendicular to the field, i.e. parallel to the plane of the electrodes. A focal conic state can also be achieved by applying only a weak electric field to a sample in its planar state. In the focal conic state the sample is scattering when viewed in normal daylight and appears bright between crossed polarisers.

A sample of an inventive compound in the different states of orientation exhibits different transmission of light. Therefore, the respective state of orientation, as well as its quality of alignment, can be controlled by measuring the light transmission of the sample depending on the strength of the applied electric field. Thereby it is also possible to determine the electric field strength required to achieve specific states of orientation and transitions between these different states. In a sample of an inventive compound of formula I, the above described focal conic state consists of many disordered birefringent small domains. By applying an electric field greater than the field for nucleation of the focal conic texture, preferably with additional shearing of the cell, a uniformly aligned texture is achieved where the helical axis is parallel to the plane of the electrodes in large, well-aligned areas. In accordance with the literature on state of the art chiral nematic materials, such as P. Rudquist et al., Liq. Cryst. 23 (4), 503 (1997), this texture is also called uniformly-lying helix (ULH) texture. This texture is required to characterize the flexoelectric properties of the inventive compound.

The sequence of textures typically observed in a sample of an inventive compound of formula I on a rubbed polyimide substrate upon increasing or decreasing electric field is given below:

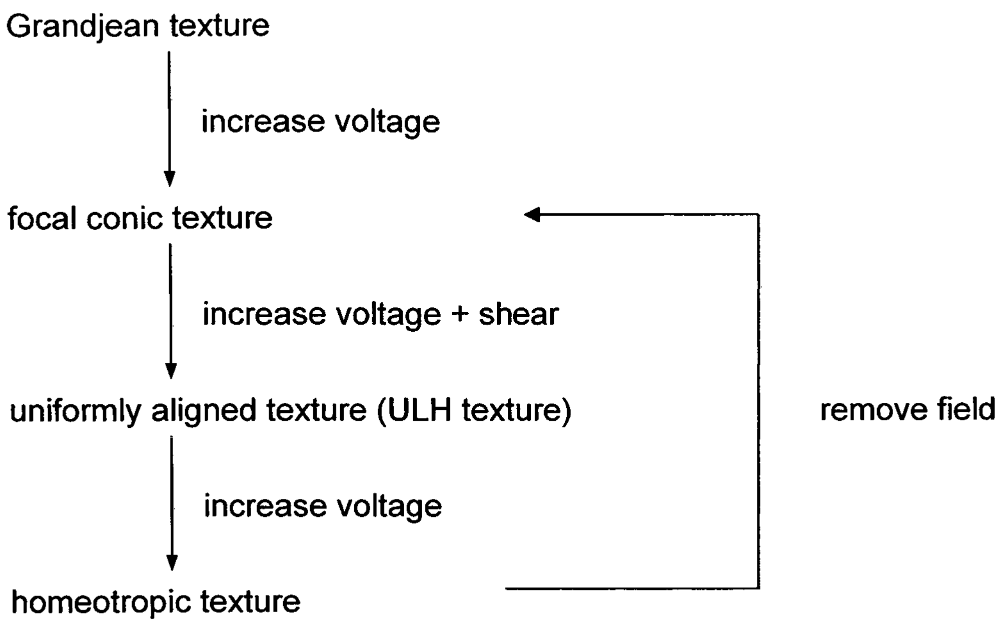


Starting from the ULH texture, the inventive flexoelectric compounds and mixtures can be subjected to flexoelectric switching by application of an electric field. This causes rotation of the optic axis of the material in the plane of the cell substrates, which leads to a change in transmission when placing the material between crossed polarisers. The flexoelectric switching of inventive materials is further described in detail in the introduction above and in the examples.
It is also possible to obtain the ULH texture, starting from the focal conic texture, by applying an electric field with a high frequency, of for example 10 kHz, to the sample whilst cooling slowly from the isotropic phase into the cholesteric phase and shearing the cell. The field frequency may differ for different compounds.

The bimesogenic compounds of formula I are particularly useful in flexoelectric liquid crystal displays as they can easily be aligned into macroscopically uniform orientation, and lead to high values of the elastic constant $k_{11}$ and a high flexoelectric coefficient e in the liquid crystal medium.

The liquid crystal medium preferably exhibits a $k_{11}$ < $1x10^{-10}$ N, preferably < $2x10^{-11}$ N, and a flexoelectric coefficient e > $1x10^{-11}$ C/m, preferably > $1x10^{-10}$ C/m.

Apart from the use in flexoelectric devices, the inventive bimesogenic compounds as well as mixtures thereof are also suitable for other types of displays and other optical and electrooptical applications, such as optical compensation or polarizing films, colour filters, reflective cholesterics, optical rotatory power and optical information storage.

A further aspect of the present invention relates to a display cell wherein the cell walls exhibit hybrid alignment conditions. The term "hybrid alignment" or orientation of a liquid crystal or mesogenic material in a display cell or between two substrates means that the mesogenic groups adjacent to the first cell wall or on the first substrate exhibit homeotropic orientation and the mesogenic groups adjacent to the second cell wall or on the second substrate exhibit planar orientation.

The term "homeotropic alignment" or orientation of a liquid crystal or mesogenic material in a display cell or on a substrate means that the mesogenic groups in the liquid crystal or mesogenic material are oriented substantially perpendicular to the plane of the cell or substrate, respectively.

The term "planar alignment" or orientation of a liquid crystal or mesogenic material in a display cell or on a substrate means that the mesogenic groups in the liquid crystal or mesogenic material are oriented substantially parallel to the plane of the cell or substrate, respectively.

A flexoelectric display according to a preferred embodiment of the present invention comprises two plane parallel substrates, preferably glass plates covered with a transparent conductive layer such as indium tin oxide (ITO) on their inner surfaces, and a flexoelectric liquid crystalline medium provided between the substrates, characterized in that one of the inner substrate surfaces exhibits homeotropic alignment conditions and the opposite inner substrate surface exhibits planar alignment conditions for the liquid crystalline medium.

Planar alignment can be achieved e.g. by means of an alignment layer, for example a layer of rubbed polyimide or sputtered $SiO_x$, that is applied on top of the substrate.

Alternatively it is possible to directly rub the substrate, i.e. without applying an additional alignment layer. For example, rubbing can be achieved by means of a rubbing cloth, such as a velvet cloth, or with a flat bar coated with a rubbing cloth. In a preferred embodiment of the present invention rubbing is achieved by means of a at least one rubbing roller, like e.g. a fast spinning roller that is brushing across the substrate, or by putting the substrate between at least two rollers, wherein in each case at least one of the rollers is optionally covered with a rubbing cloth. In another preferred

embodiment of the present invention rubbing is achieved by wrapping the substrate at least partially at a defined angle around a roller that is preferably coated with a rubbing cloth.

Homeotropic alignment can be achieved e.g. by means of an alignment layer coated on top of the substrate. Suitable aligning agents used on glass substrates are for example alkyltrichlorosilane or lecithine, whereas for plastic substrate thin layers of lecithine, silica or high tilt polyimide orientation films as aligning agents may be used. In a preferred embodiment of the invention silica coated plastic film is used as a substrate.

Further suitable methods to achieve planar or homeotropic alignment are described for example in J. Cognard, Mol. Cryst. Liq. Cryst. 78, Supplement 1, 1-77 (1981).

By using a display cell with hybrid alignment conditions, a very high switching angle of flexoelectric switching, fast response times and a good contrast can be achieved.

The flexoelectric display according to present invention may also comprise plastic substrates instead of glass substrates. Plastic film substrates are particularly suitable for rubbing treatment by rubbing rollers as described above.

Another object of the present invention is that compounds of formula I, when added to a nematic liquid crystalline mixture, produce a phase below the nematic.

Accordingly, the bimesogenic compounds of formula I according to the present invention allow the second nematic phase to be induced in nematic mixtures that do not show evidence of this phase normally. Furthermore, varying the amounts of compounds of formula I allow the phase behaviour of the second nematic to be tailored to the required temperature.

Examples for this are given and the mixtures obtainable thereof are particularly useful for flexoelectric liquid crystal display. Thus, another object of the present invention is liquid crystal media comprising one or more compounds of formula I exhibiting a second nematic phase.

Without further elaboration, it is believed that one skilled in the art can, using the preceding description, utilize the present invention to its fullest extent. The following examples are, therefore, to be construed as merely illustrative and not limitative of the remainder of the disclosure in any way whatsoever.

Unless the context clearly indicates otherwise, as used herein plural forms of the terms herein are to be construed as including the singular form and vice versa.

Throughout the description and claims of this specification, the words "comprise" and "contain" and variations of the words, for example "comprising" and "comprises", mean "including but not limited to", and are not intended to (and do not) exclude other components.

Herein it is to be understood that the angles of the bonds at a C atom being bound to three adjacent atoms, e.g. in a C=C or C=O double bond or e.g. in a benzene ring, are 120° and that the angles of the bonds at a C atom being bound to two adjacent atoms, e.g. in a C≡C or in a C≡N triple bond or in an allylic position C=C=C are 180°, unless these angles are otherwise restricted, e.g. like being part of small rings, like 3-, 5- or 5-atomic rings, notwithstanding that in some instances in some structural formulae these angles are not represented exactly.

It will be appreciated that variations to the foregoing embodiments of the invention can be made while still falling within the scope of the invention. Each feature disclosed in this specification, unless stated otherwise, may be replaced by alternative features serving the same, equivalent or similar purpose. Thus, unless stated otherwise, each feature disclosed is one example only of a generic series of equivalent or similar features.

All of the features disclosed in this specification may be combined in any combination, except combinations where at least some of such features and/or steps are mutually exclusive. In particular, the preferred features of the invention are applicable to all aspects of the invention and may be used in any combination. Likewise, features described in non-essential combinations may be used separately (not in combination).

The total concentration of all compounds in the media according to the present invention is 100 %.

In the foregoing and in the following examples, unless otherwise indicated, all temperatures are set forth uncorrected in degrees Celsius and all parts and percentages are by weight.

The following abbreviations are used to illustrate the liquid crystalline phase behaviour of the compounds: K = crystalline; N = nematic; N2 = second nematic; S or Sm = smectic; Ch = cholesteric; I = isotropic; Tg = glass transition. The numbers between the symbols indicate the phase transition temperatures in °C.

Herein, the structures of the liquid crystal compounds are represented by abbreviations, which are also called "acronyms". The transformation of the abbreviations into the corresponding structures is straight forward according to the following three tables A to C.

All groups $C_nH_{2n+1}$, $C_mH_{2m+1}$, and $C_lH2_{l+1}$ are preferably straight chain alkyl groups with n, m and l C-atoms, respectively, all groups $C_nH_{2n}$, $C_mH_{2m}$ and $C_lH_{2l}$ are preferably $(CH_2)_n$, $(CH_2)_m$ and $(CH_2)_l$, respectively and -CH=CH- preferably is *trans*- respectively *E* vinylene.

Table A lists the symbols used for the ring elements, table B those for the linking groups and table C those for the symbols for the left hand and the right hand end groups of the molecules.

Table D lists exemplary molecular structures together with their respective codes.

**Table A: Ring Elements**

| | | | |
|---|---|---|---|
| **C** | 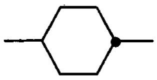 | **P** | 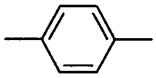 |
| **D** | 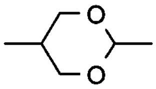 | **DI** | 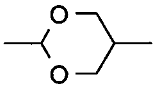 |
| **A** | 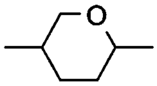 | **AI** | 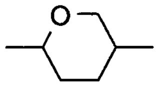 |
| **G** | 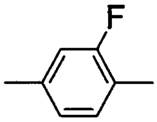 | **GI** | 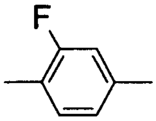 |
| **G(Cl)** | 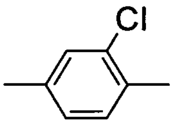 | **GI(Cl)** | 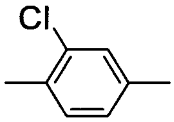 |
| **G(1)** | 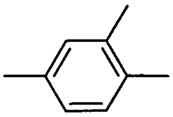 | **GI(1)** | 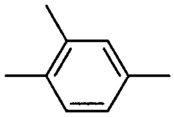 |
| **U** | 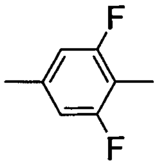 | **UI** | 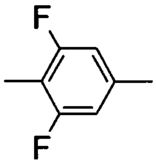 |
| **Y** | 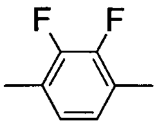 | | |
| **M** | 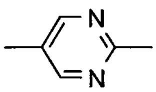 | **MI** | 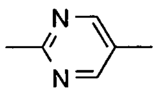 |
| **N** | 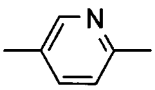 | **NI** | 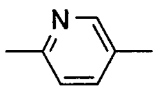 |
| **np** | 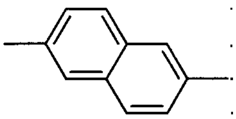 | | |
| **n3f** | 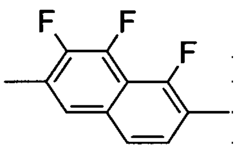 | **n3fI** | 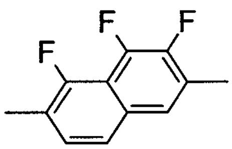 |
| **th** | 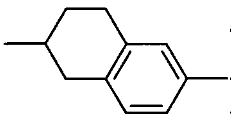 | **thI** | 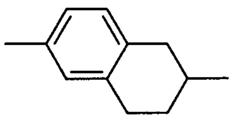 |

(continued)

| | | | |
|---|---|---|---|
| th2f | 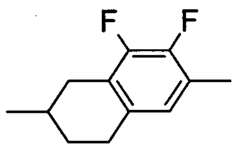 | th2fl | 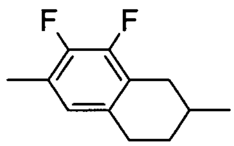 |
| o2f | 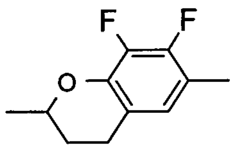 | o2fl | 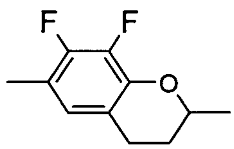 |
| dh | 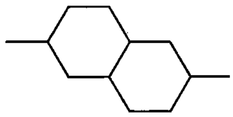 | | |
| K | 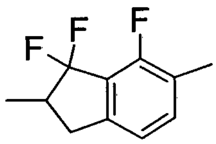 | Kl | 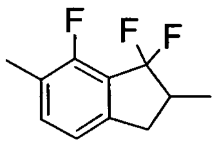 |
| L | 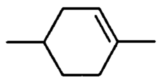 | Ll | 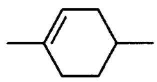 |
| F | 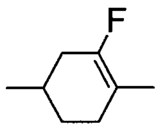 | Fl | 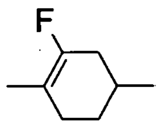 |

**Table B: Linking Groups**

| | | | |
|---|---|---|---|
| **n** | $(-CH_2-)_n$ | "n" is an integer except 0 and 2 | |
| **E** | $-CH_2-CH_2-$ | | |
| **V** | -CH=CH- | | |
| **T** | -C≡C- | | |
| **W** | $-CF_2-CF_2-$ | | |
| **B** | -CF=CF- | | |
| **Z** | -CO-O- | **ZI** | -O-CO- |
| **X** | -CF=CH- | **XI** | -CH=CF- |
| **1O** | $-CH_2-O-$ | **O1** | $-O-CH_2-$ |
| **Q** | $-CF_2-O-$ | **QI** | $-O-CF_2-$ |

**Table C: End Groups**

| **Left hand side, used alone or in combination with others** | | **Right hand side, used alone or in combination with others** | |
|---|---|---|---|
| **-n-** | $C_nH_{2n+1}-$ | **-n** | $-C_nH_{2n+1}$ |
| **-nO-** | $C_nH_{2n+1}-O-$ | **-nO** | $-O-C_nH_{2n+1}$ |
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH-C_nH_{2n}-$ | **-Vn** | $-CH=CH-C_nH_{2n+1}$ |
| **-nVm-** | $C_nH_{2n+1}-CH=CH-C_mH_{2m}-$ | **-nVm** | $-C_nH_{2n}-CH=CH-C_mH_{2m+1}$ |
| **-N-** | N≡C- | **-N** | -C≡N |

(continued)

| Left hand side, used alone or in combination with others | | Right hand side, used alone or in combination with others | |
|---|---|---|---|
| **-S-** | S=C=N- | **-S** | -N=C=S |
| **-F-** | F- | **-F** | -F |
| **-CL-** | Cl- | **-CL** | -Cl |
| **-M-** | $CFH_2$- | **-M** | -$CFH_2$ |
| **-D-** | $CF_2H$- | **-D** | -$CF_2H$ |
| **-T-** | $CF_3$- | **-T** | -$CF_3$ |
| **-MO-** | $CFH_2O$- | **-OM** | -$OCFH_2$ |
| **-DO-** | $CF_2HO$- | **-OD** | -$OCF_2H$ |
| **-TO-** | $CF_3O$- | **-OT** | -$OCF_3$ |
| **-A-** | H-C≡C- | **-A** | -C≡C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |
| **Left hand side, used in combination with others only** | | **Right hand side, used in combination with others only** | |
| **-...n...-** | $(-CH_2-)_n$ | **-...n...** | $(-CH_2-)_n$ |
| **-...M...-** | -CFH- | **-...M...** | -CFH- |
| **-...D...-** | -$CF_2$- | **-...D...** | -$CF_2$- |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

wherein n und m each are integers and three points "..." indicate a space for other symbols of this table.

Preferably the liquid crystalline media according to the present invention comprise, besides the compound(s) of formula I one or more compounds selected from the group of compounds of the formulae of the following table.

<u>Table D</u>

In this table n is an integer selected from 3 and 5 to 15, preferably from 3, 5, 7 and 9, unless explicitly defined otherwise.

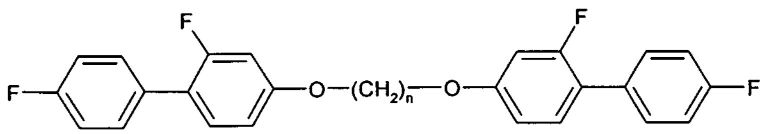

**F-PGI-O-n-O-GP-F**

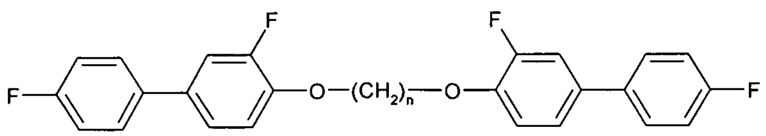

**F-PG-O-n-O-GIP-F**

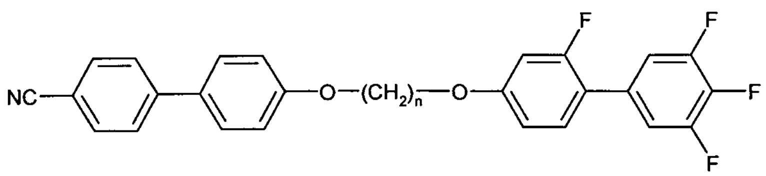

**N-PP-O-n-O-GU-F**

(continued)

In this table n is an integer selected from 3 and 5 to 15, preferably from 3, 5, 7 and 9, unless explicitly defined otherwise.

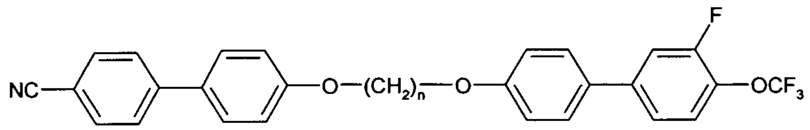

**N-PP-O-n-O-PG-OT**

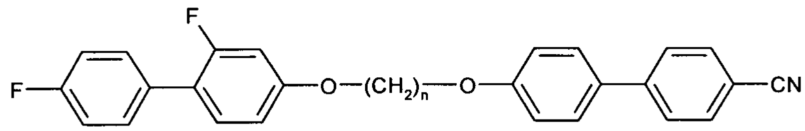

**F-PGI-O-n-O-PP-N**

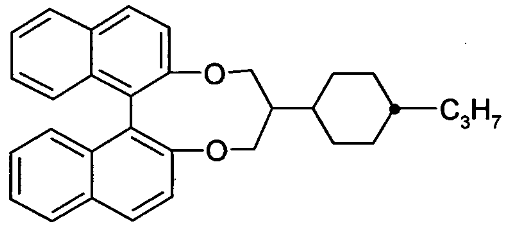

**R-5011** respectively **S-5011**

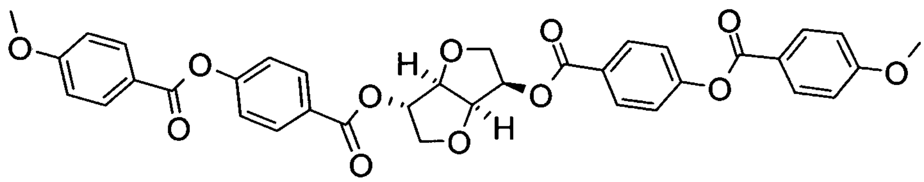

**CD-1**

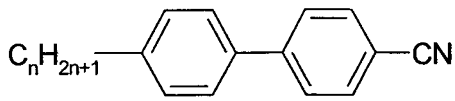

**PP-n-N** ($n \in \{2; 3; 4; 5; 6; 7\}$)

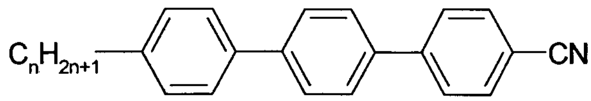

**PPP-n-N** ($n \in \{2; 3; 4; 5; 6; 7\}$)

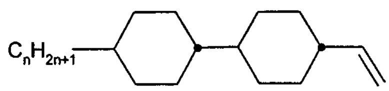

**CC-n-V** ($n \in \{2; 3; 4; 5; 7\}$)

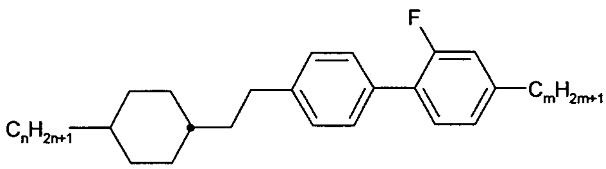

**CEPGI-n-m** ($n \in \{2; 3; 4; 5\}$; $m \in \{2; 3; 4; 5\}$)

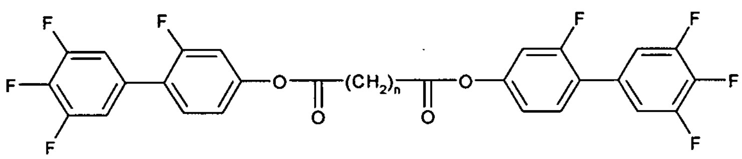

(continued)

In this table n is an integer selected from 3 and 5 to 15, preferably from 3, 5, 7 and 9, unless explicitly defined otherwise.

**F-UIGI-ZI-n-Z-GU-F**

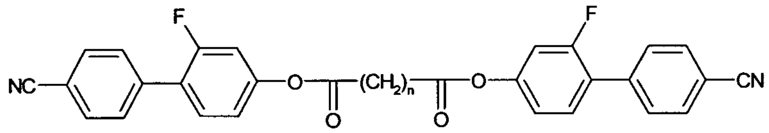

**N-PGI-ZI-n-Z-GP-N**

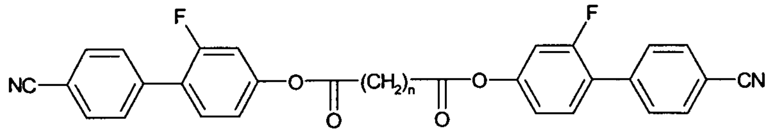

**F-PGI-ZI-n-Z-GP-F**

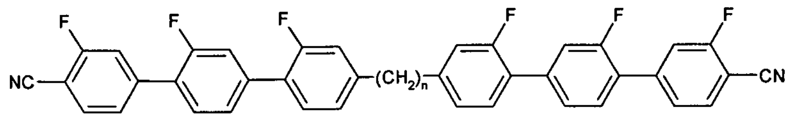

**N-GIGIGI-n-GGG-N**

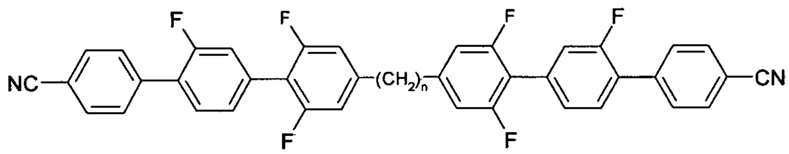

**N-PGIUI-n-UGP-N**

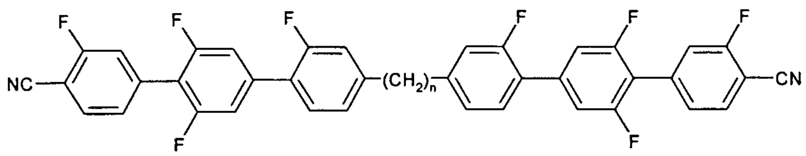

**N-GIUIGI-n-GUG-N**

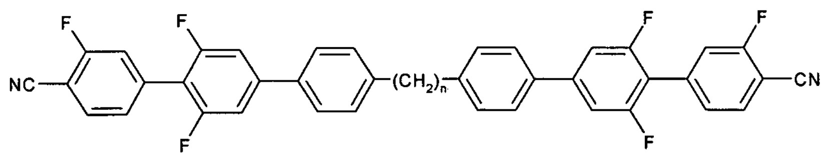

**N-GIUIP-n-PUG-N**

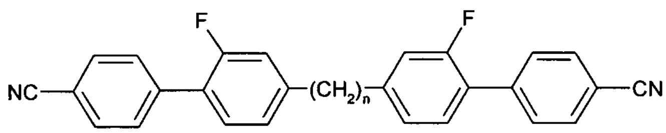

**N-PGI-n-GP-N**

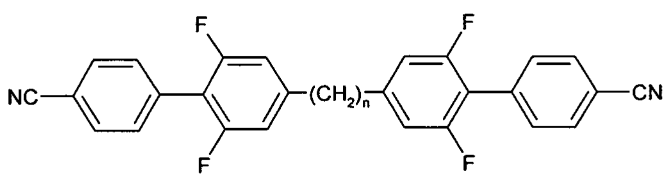

**N-PUI-n-UP-N**

(continued)

In this table n is an integer selected from 3 and 5 to 15, preferably from 3, 5, 7 and 9, unless explicitly defined otherwise.

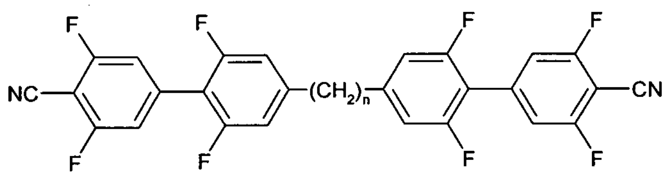

**N-UIUI-n-UU-N**

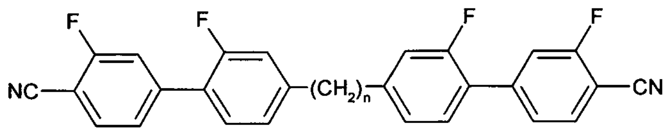

**N-GIGI-n-GG-N**

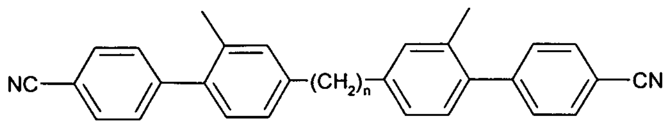

**N-PGI(1 )-n-G(1)P-N**

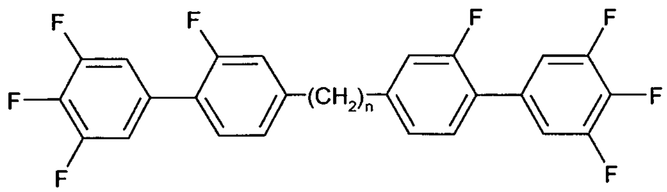

**F-UIGI-n-GU-F**

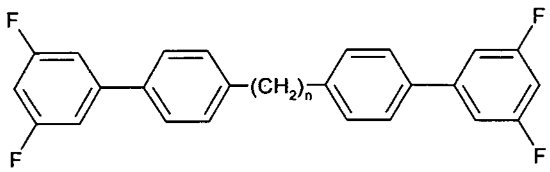

**UIP-n-PU**

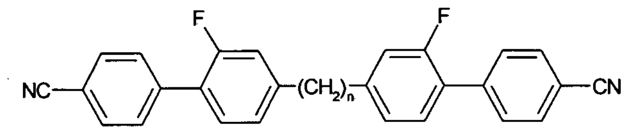

**N-PGI-n-GP-N**

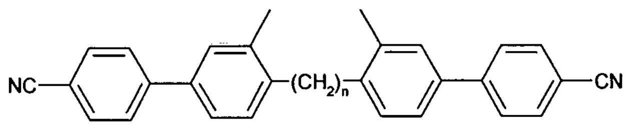

**N-PG(1 )-n-GI(1)P-N**

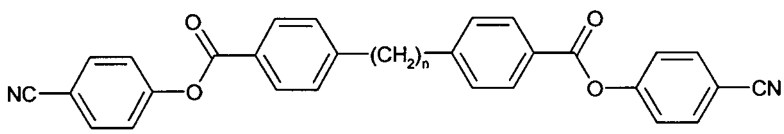

**N-PZIP-n-PZP-N**

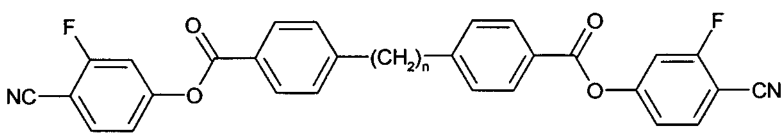

(continued)

In this table n is an integer selected from 3 and 5 to 15, preferably from 3, 5, 7 and 9, unless explicitly defined otherwise.

**N-GIZIP-n-PZG-N**

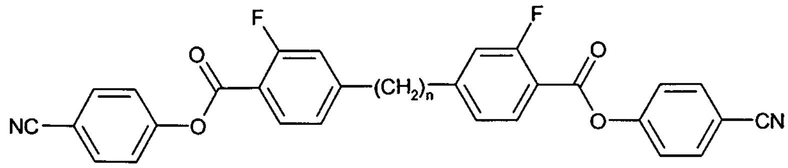

**N-PZIGI-n-GZP-N**

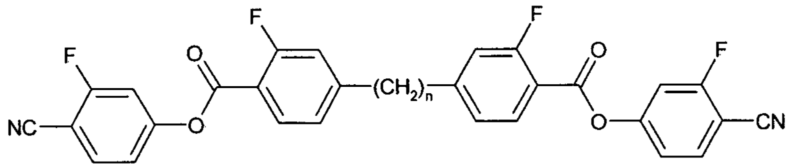

**N-GIZIGI-n-GZG-N**

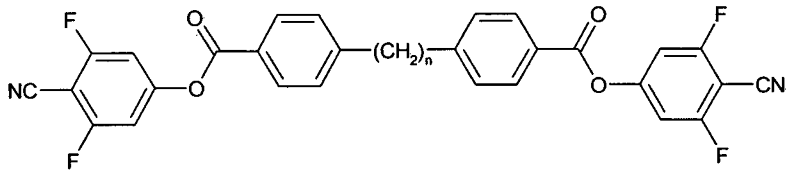

**N-UIZIP-n-PZU-N**

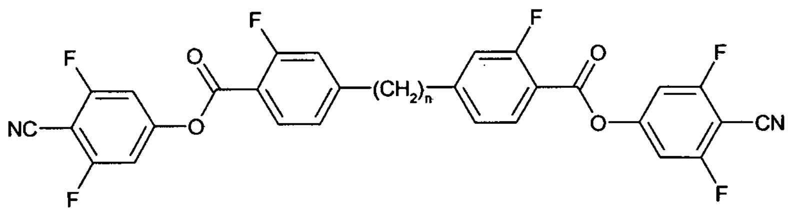

**N-UIZIGI-n-GZU-N**

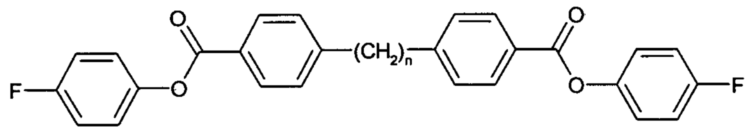

**F-PZIP-n-PZP-F**

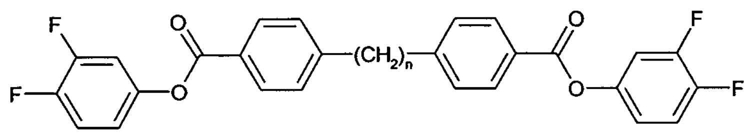

**F-GIZIP-n-PZG-F**

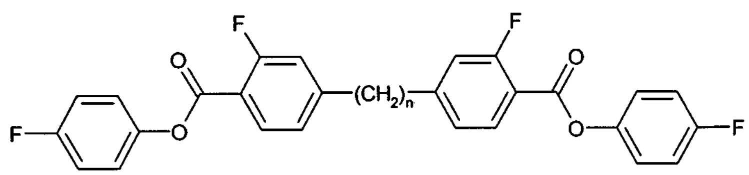

**F-PZIGI-n-GZP-F**

(continued)

In this table n is an integer selected from 3 and 5 to 15, preferably from 3, 5, 7 and 9, unless explicitly defined otherwise.

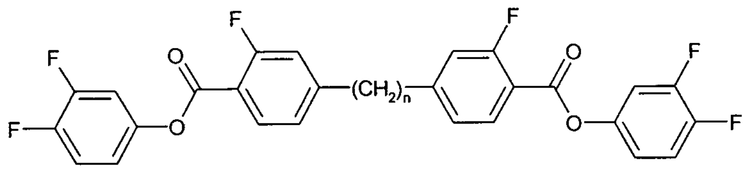

**F-GIZIGI-n-GZG-F**

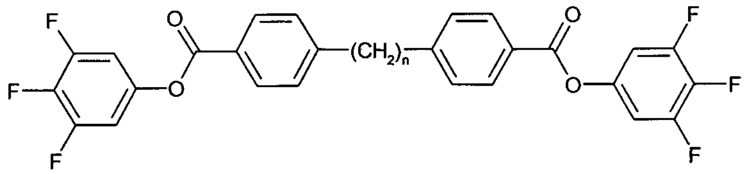

**F-UIZIP-n-PZU-F**

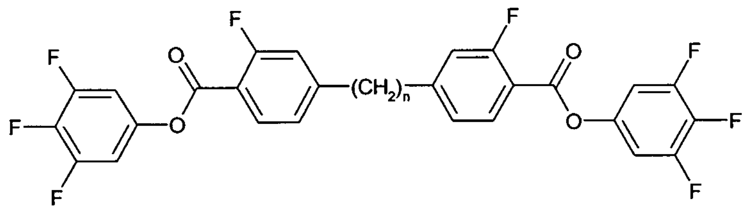

**F-UIZIGI-n-GZU-F**

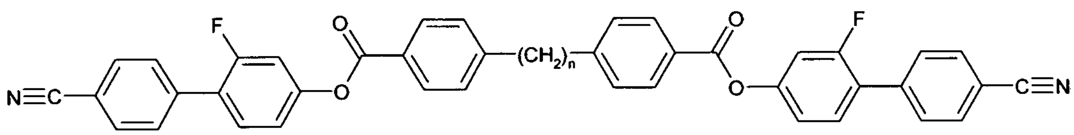

**N-PGIZIP-n-PZGP-N**

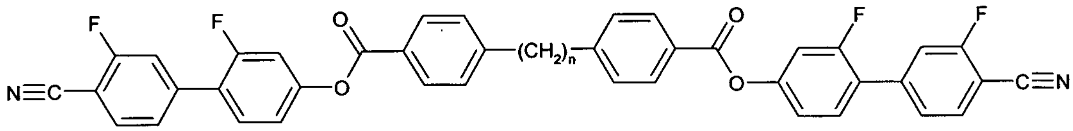

**N-GIGIZIP-n-PZGG-N**

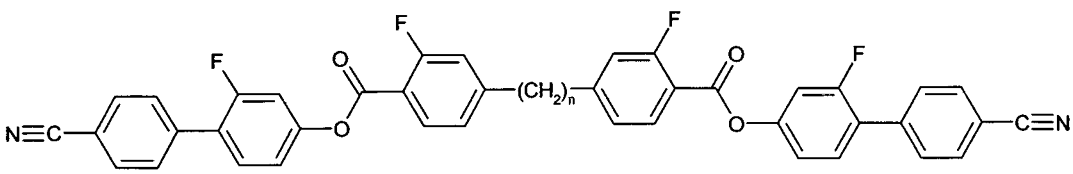

**N-PGIZIGI-n-GZGP-N**

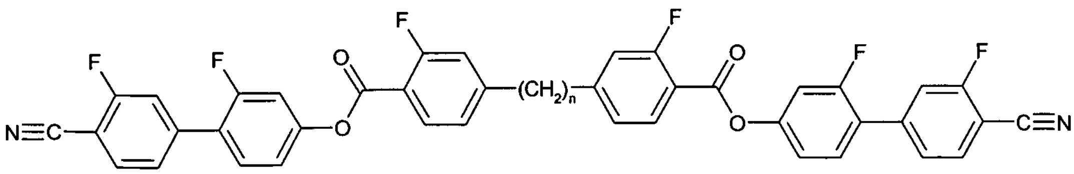

**N-GIGIZIGI-n-GZGG-N**

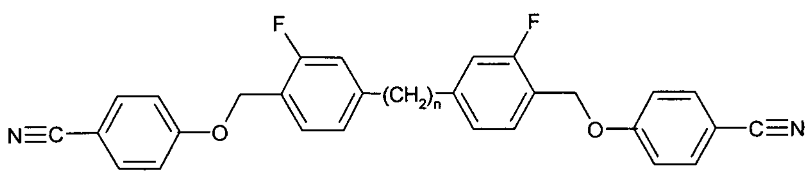

(continued)

In this table n is an integer selected from 3 and 5 to 15, preferably from 3, 5, 7 and 9, unless explicitly defined otherwise.

**N-PO1 GI-n-GO1 P-N**

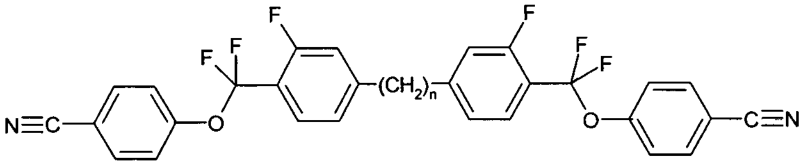

**N-PQIGI-n-GQP-N**

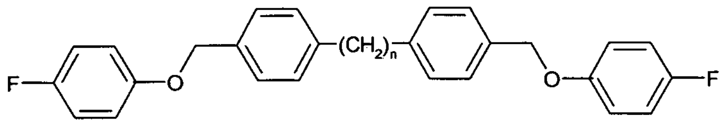

**F-PO1P-n-PO1P-N (= N-PO1P-n-PO1P-F)**

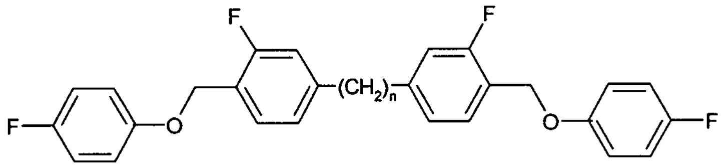

**F-PO1GI-n-GO1P-N (= N-PO1GI-n-GO1P-F)**

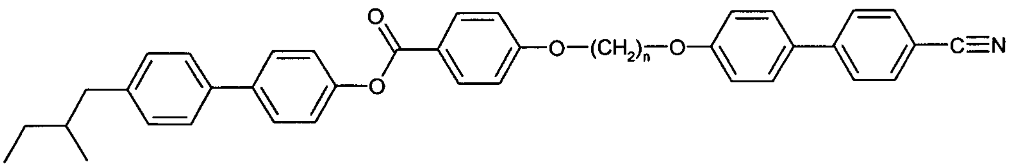

**2(CHCH3)1-PPZIP-O-n-O-PP-N**

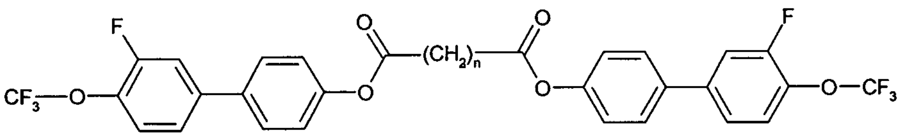

**TO-GIP-ZI-n-Z-PG-OT**

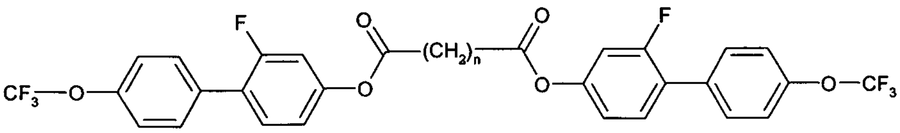

**TO-PGI-ZI-n-Z-GP-OT**

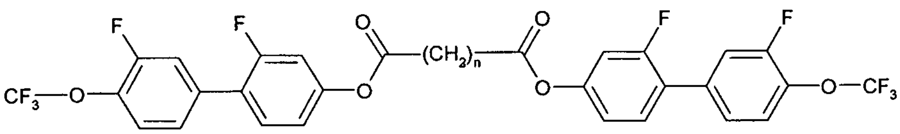

**TO-GIGI-ZI-n-Z-GG-OT**

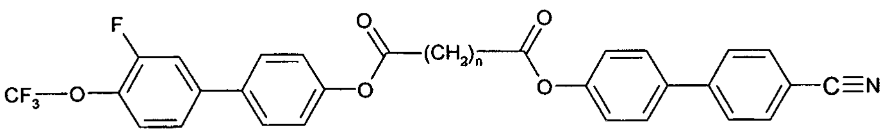

**TO-GIP-ZI-n-Z-PP-N**

(continued)

In this table n is an integer selected from 3 and 5 to 15, preferably from 3, 5, 7 and 9, unless explicitly defined otherwise.

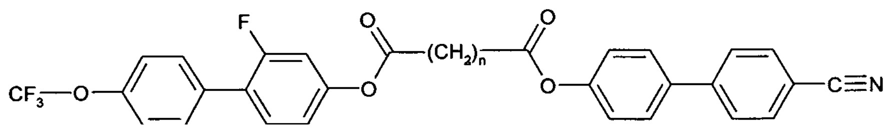

**TO-PGI-ZI-n-Z-PP-N**

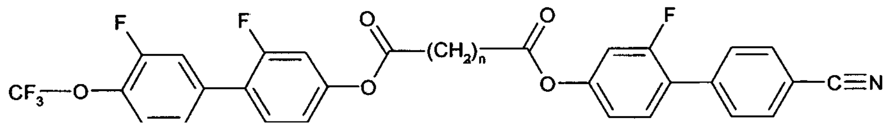

**TO-GIGI-ZI-n-Z-GP-N**

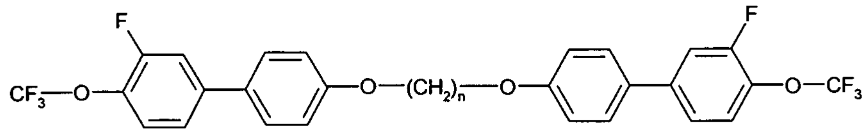

**TO-GIP-O-n-O-PG-OT**

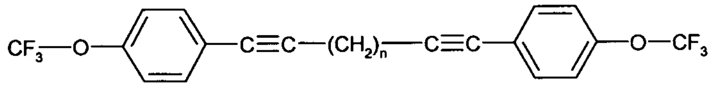

**TO-P-T-n-T-P-OT**

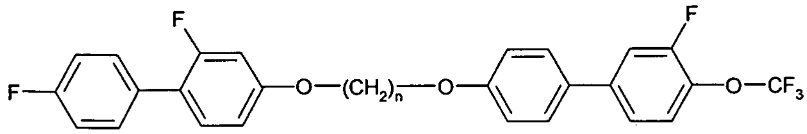

**F-PGI-O-n-O-PG-OT**

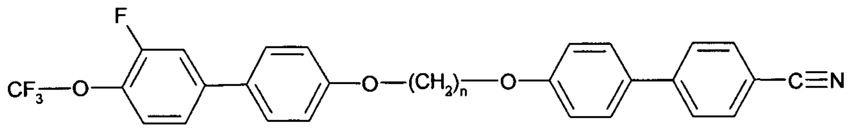

**TO-GIP-O-n-O-PP-N**

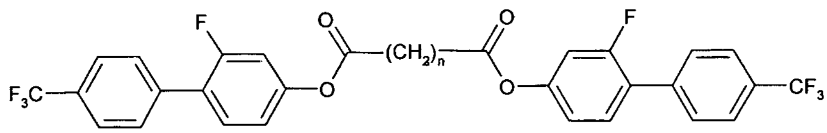

**T-PGI-ZI-n-Z-GP-T**

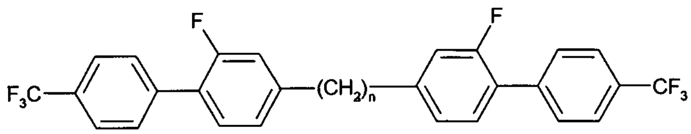

**T -PGI-n-GP-T**

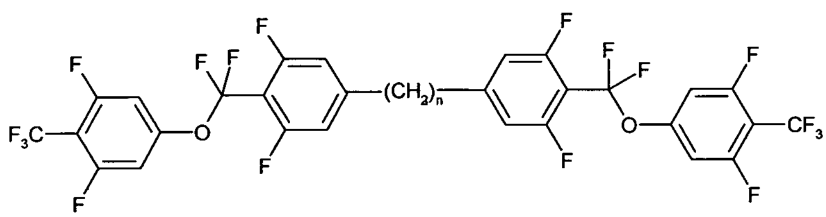

**T-UIQIUI-n-UQU-T**

(continued)

In this table n is an integer selected from 3 and 5 to 15, preferably from 3, 5, 7 and 9, unless explicitly defined otherwise.

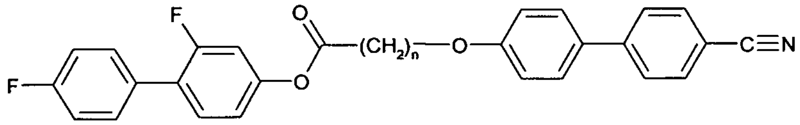

**F-PGI-ZI-n-O-PP-N**

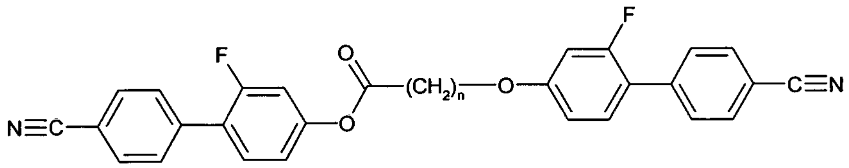

**N-PGI-ZI-n-O-GP-N**

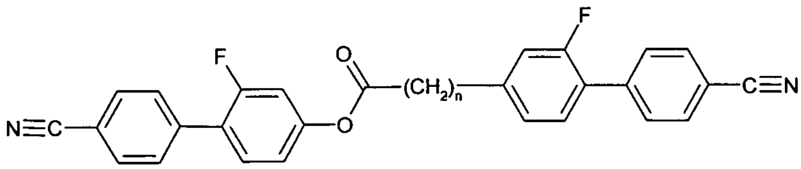

**N-PGI-ZI-n-GP-N**

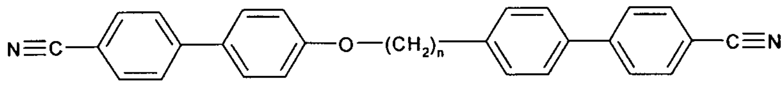

**N-PP-ZI-n-PP-N**

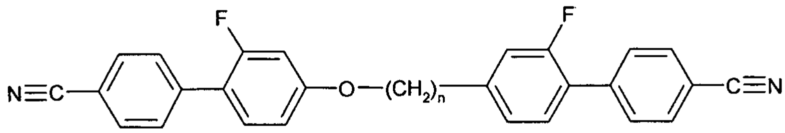

**N-PGI-O-n-GP-N**

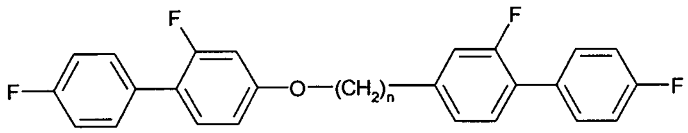

**F-PGI-O-n-GP-F**

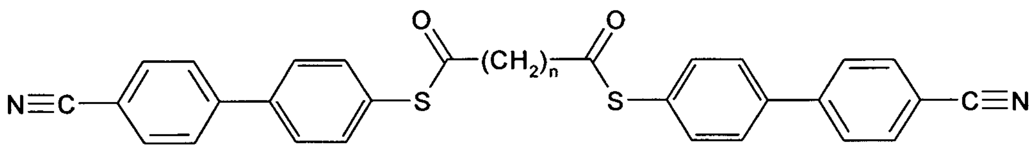

**N-PP-SCO-n-COS-PP-N**

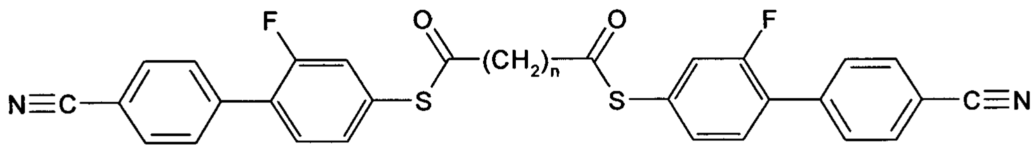

**N-PGI-SCO-n-COS-GP-N**

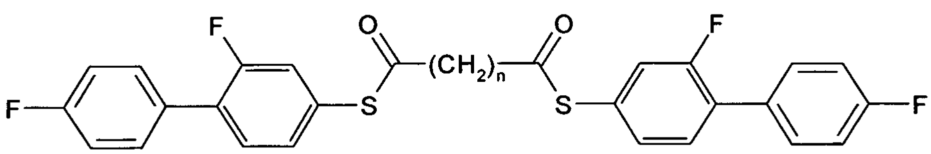

**F-PGI-SCO-n-COS-GP-F**

(continued)

In this table n is an integer selected from 3 and 5 to 15, preferably from 3, 5, 7 and 9, unless explicitly defined otherwise.

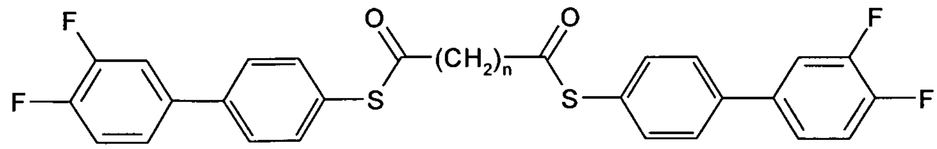

**F-GIP-SCO-n-COS-PG-F**

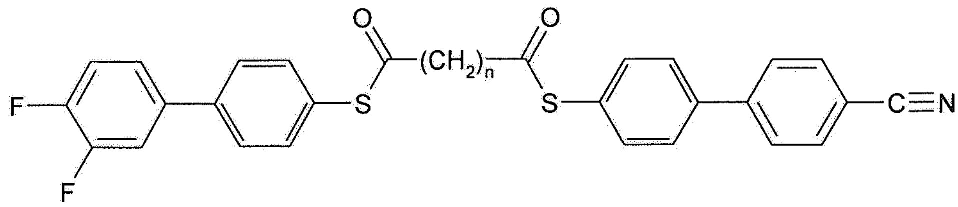

**F-GIP-SCO-n-COS-PP-N**

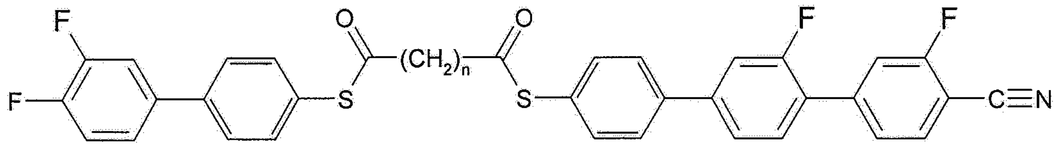

**F-GIP-SCO-n-COS-PGG-N**

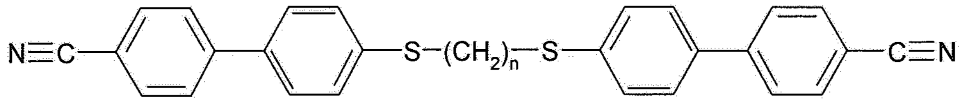

**N-PP-S-9-S-PP-N**

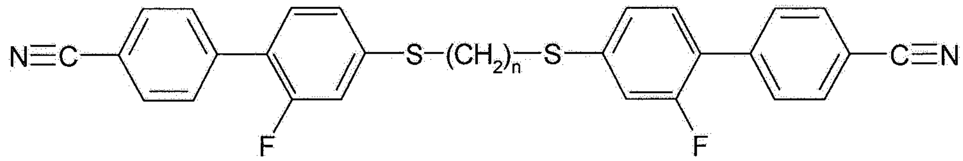

**N-PGI-S-n-S-GP-N**

wherein n is an integer from 12t o 15, preferably an even integer.

Further preferred co-components of the medium according to the invention are those abbreviated by the following acronyms:

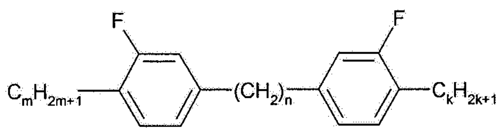

**m-P-n-P-k**

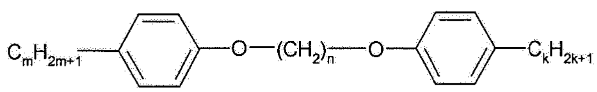

**m-P-O-n-O-P-k**

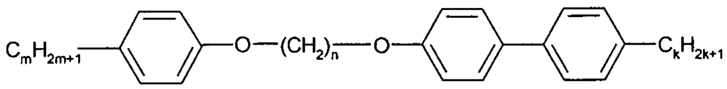

**m-P-O-n-O-PP-k**

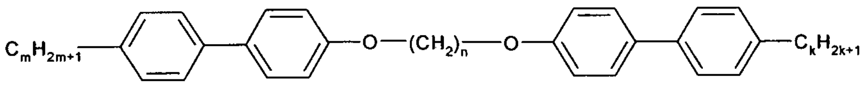

**m-PP-O-n-O-PP-k**

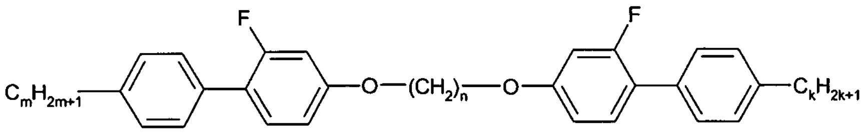

**m-PGI-O-n-O-GP-k**

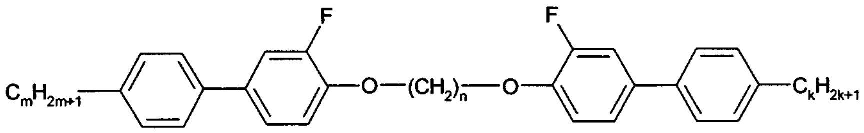

**m-PG-O-n-O-GIP-k**

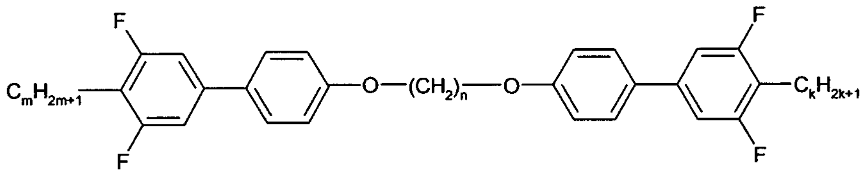

**m-UIP-O-n-O-PU-k**

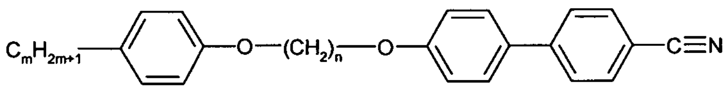

**m-P-O-n-O-PP-N**

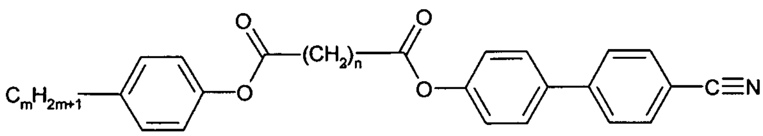

**m-P-ZI-n-Z-PP-N**

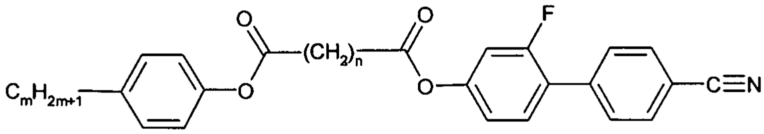

**m-P-ZI-n-Z-GP-N**

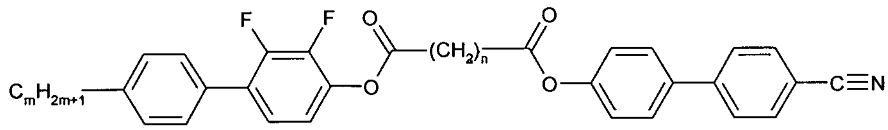

m-PY-ZI-n-Z-PP-N

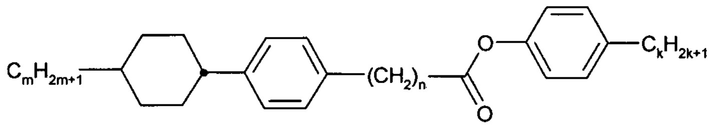

m-CP-n-Z-P-k

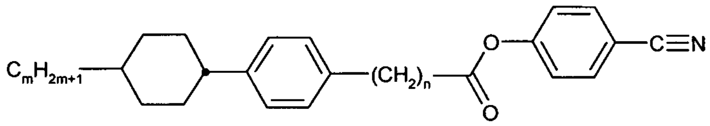

m-CP-n-Z-P-N

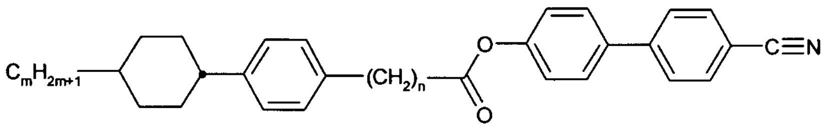

m-CP-n-Z-PP-N

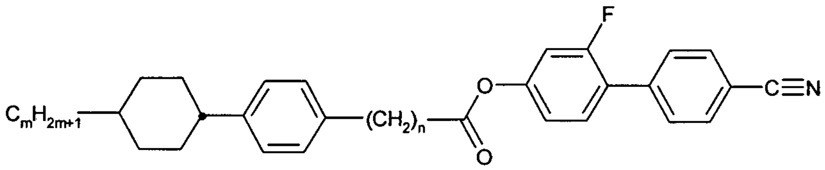

m-CP-n-Z-GP-N

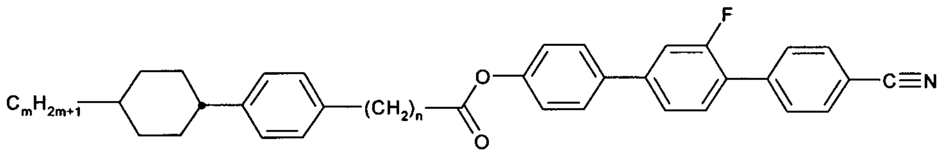

m-CP-n-Z-PGP-N

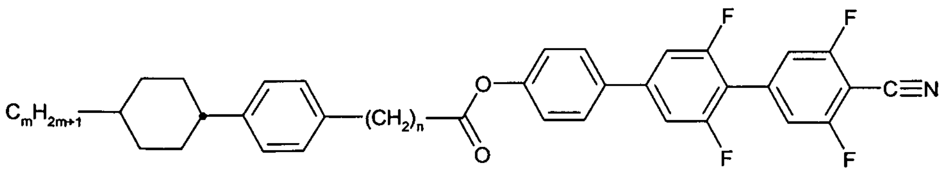

m-CP-n-Z-PUU-N

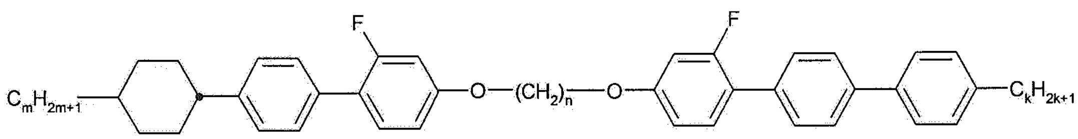

m-CPGI-O-n-O-GPP-k

wherein m and k are independently of each other an integer from 1 to 9 preferably from 1 to 7, more preferably from 3 to 5 and n is an integer from 1 to 15, preferably an odd integer from 3 to 9; specific examples are the compounds with the following acronyms.

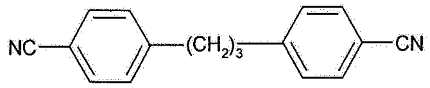

N-P-3-P-N

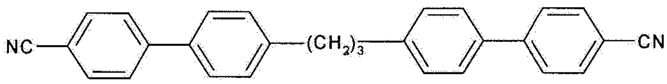

N-PP-3-PP-N

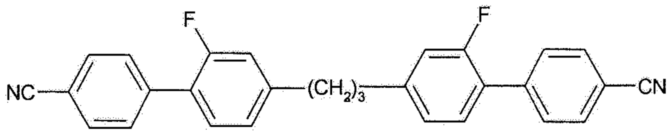

N-PGI-3-GP-N

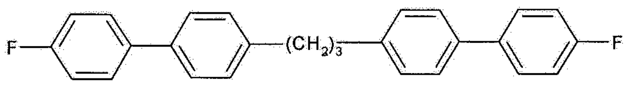

F-PP-3-PP-F

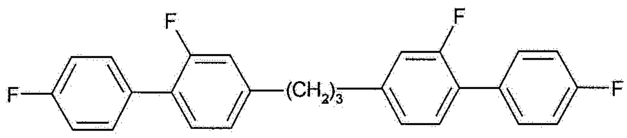

F-PGI-3-GP-F

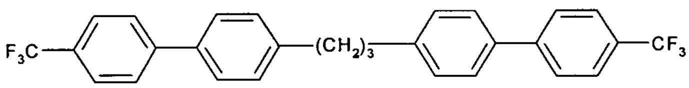

T-PP-3-PP-T

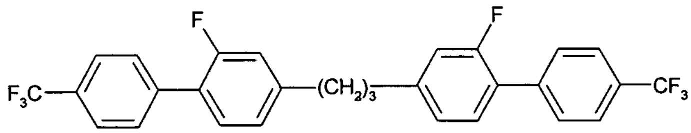

T-PGI-3-GP-T

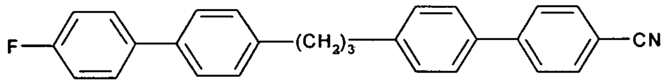

F-PP-3-PP-N

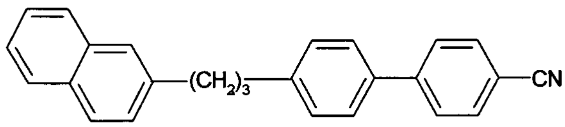

np-3-PP-N

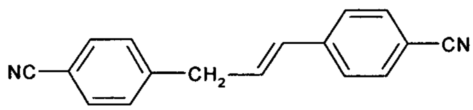

N-P-1-V-P-N

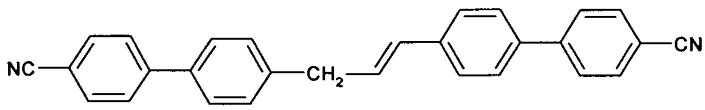

N-PP-1-V-PP-N

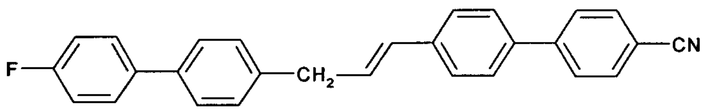

F-PP-1-V-PP-N

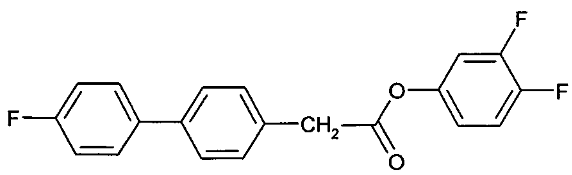

F-PP-1-Z-G-F

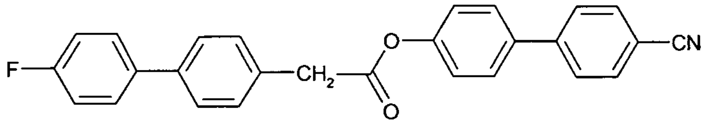

F-PP-1-Z-PP-N

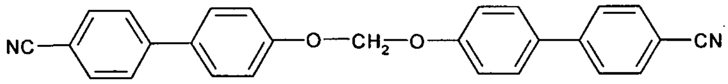

N-PP-O-1-O-PP-N

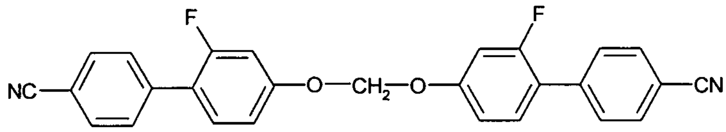

N-PGI-O-1-O-GP-N

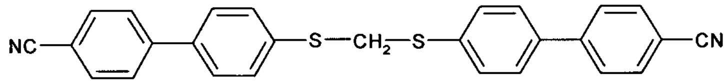

N-PP-S-1-S-PP-N

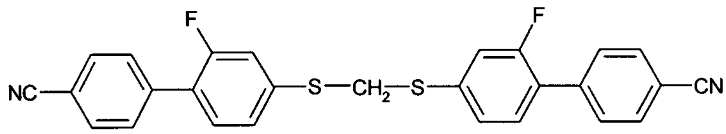

N-PGI-S-1-S-GP-N

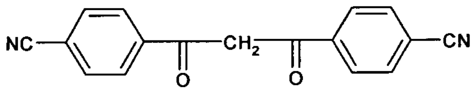

N-P-K-1-K-P-N

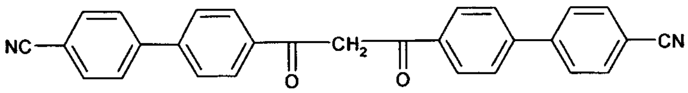

N-PP-K1K-PP-N

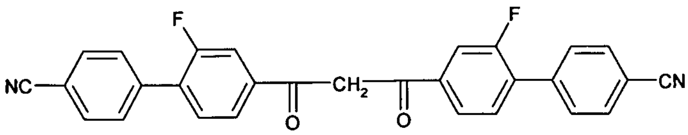

N-PGI-K1K-GP-N

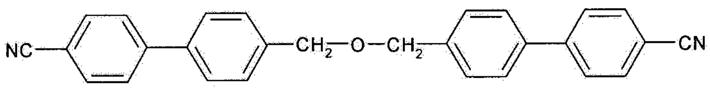

N-PP-1O1-PP-N

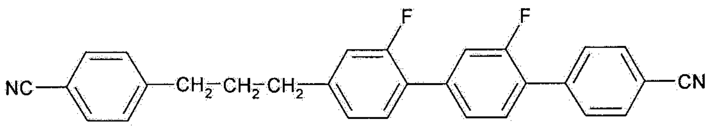

N-P-3-GGP-N .

Preferred compounds of formula I used in the medium according to the present invention are those abbreviated by the following acronyms:

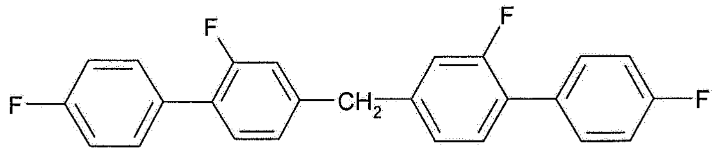

**F-PGI-1-GP-F**

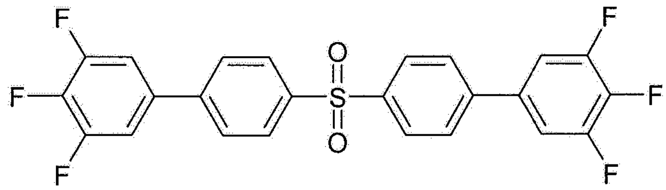

**F-UIP-(SO2)-PU-F**

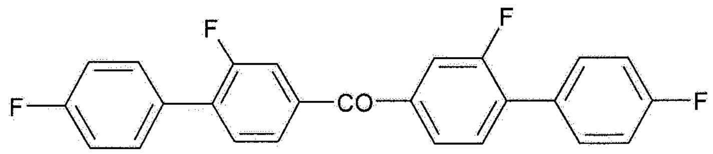

**F-PGI-K-GP-F .**

Syntheses of compounds of formula I

Compound 1: Preparation of:

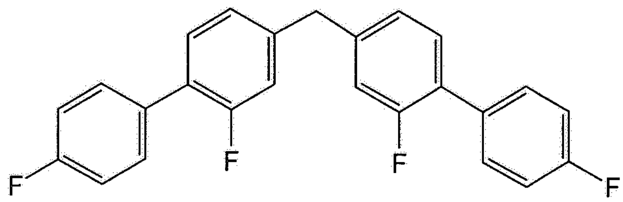

The compound of interest is prepared according to the following scheme.

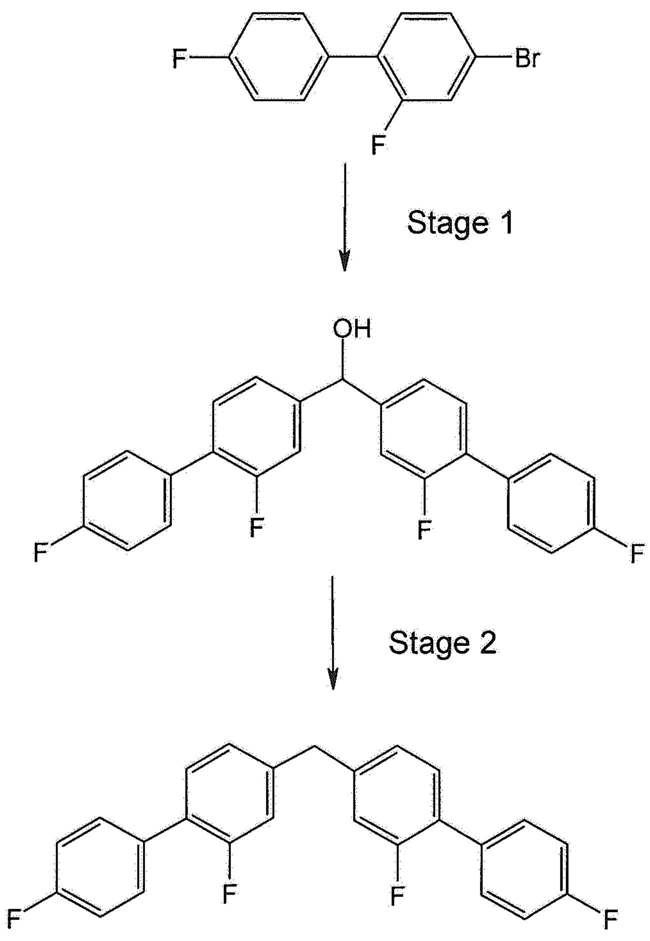


Stage 1.1

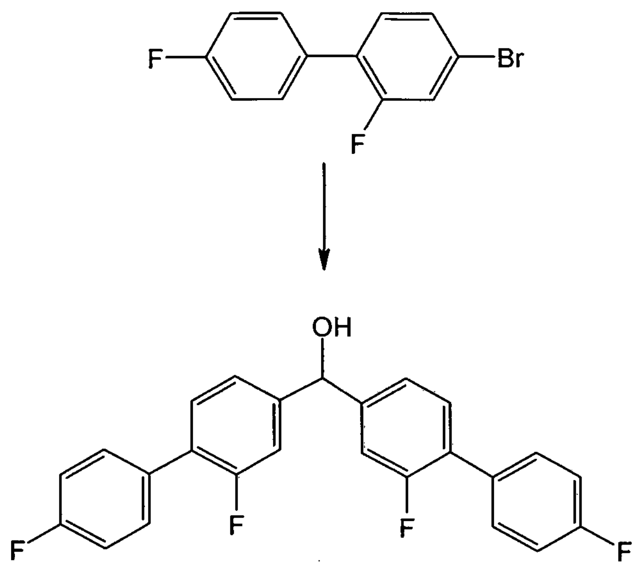

Magnesium turnings (1.3 g, 54 mmol) and tetrahydrofuran (THF, 2 mL) are placed in an ultrasonic bath under nitrogen (a procedure, which is called short "ultrasonicated" or "sonicated" in this application) for 30 minutes. Iodine (0.15 g, 0.6 mmol) is added and the mixture is heated until the colour of the iodine disappears. 4-Bromo-2,4'difluorobiphenyl (13.1 g, 50 mmol) dissolved in THF (20 mL) is charged into a pressure equalizing funnel. Approximately five percent thereof are added and the mixture is heated under reflux until the Grignard reaction has started. The remainder of the 4-bromo-2,4'-difluorobiphenyl is added over a time span of 10 minutes under reflux and then the mixture is heated

under reflux for a further 2 hours. Then the mixture is cooled to 20°C. Ethyl formate (2 mL, 25 mmol) is added dropwise over a time span of five minutes at a temperature in the range from 20°C to 30°C. Then the mixture is stirred for a further 1.5 hours. Subsequently the mixture is acidified with concentrated sulphuric acid (5 mL) and water (75 mL). The mixture is extracted with dichloromethane (DCM, $CH_2Cl_2$, 1 x 200 mL and 2 x 50 mL). The organic layer is dried over anhydrous sodium sulphate, filtered, washed with DCM and the solvent from the filtrate is removed *in vacuo.* The residue is purified by vacuum flash chromatography on silica (50 g) eluting with the following mixtures DCM:Petrol (B.p. 40:60), 50:50, 60:40, 70:30, 80:20, 90:10, then 100:0 mL:mL. The fractions containing the product are collected and the solvent is removed *in vacuo.* The solid is triturated with petrol (B.p. 40:60) to give the desired product.

Stage 1.2

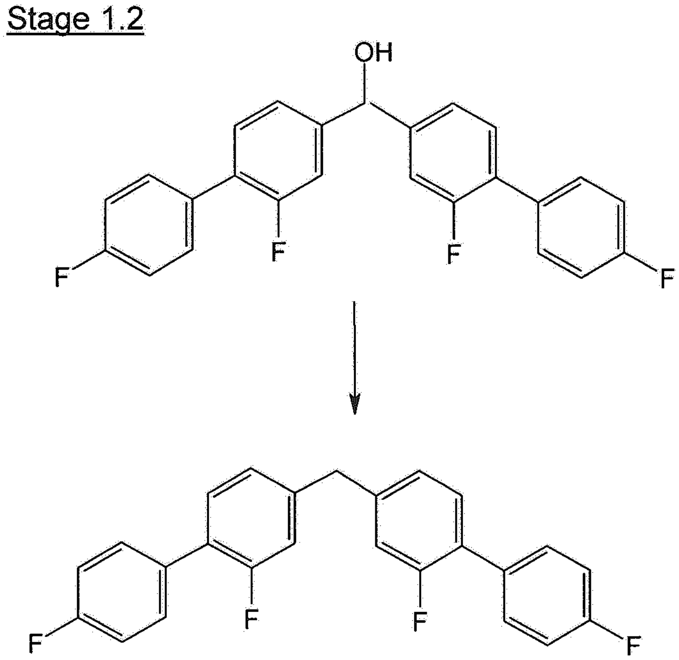

The product from stage 1 (5 g, 12.2 mmol) is dissolved in THF (100 mL) and 10% palladium on carbon (1 g) are added. The material is hydrogenated at ambient temperature (also called room temperature, short RT), which is 20°C herein, unless explicitly specified otherwise, for 16 hours. TLC shows no reaction. The catalyst is filtered off and the solvent from the filtrate removed *in vacuo.* The residue is dissolved in THF (100 mL) and 20 % palladium hydroxide (1 g) added. The mixture is hydrogenated at 30°C for 5 hours and then kept for 16 hours at room temperature. Glacial acetic acid (20 mL) is added and the mixture is hydrogenated for a further 5 hours at 40°C. The catalyst is filtered off and the solvent from the filtrate removed *in vacuo.* The residue is purified by vacuum flash chromatography on silica (50 g) eluting with the following mixtures toluene: Petrol (B.p. 40:60) 20:80, 30:70, 40:60, 50:50, 60:40, 70:30, 80:20 mL/mL. The fractions containing the product are collected and the solvent removed *in vacuo.* The solid is re-crystallized from acetonitrile (15 mL) to give the desired product.

The product has the following properties. Phase sequence: K 70.9 I, e/K = 1.97 $V^{-1}$.

Compound 2: Preparation of

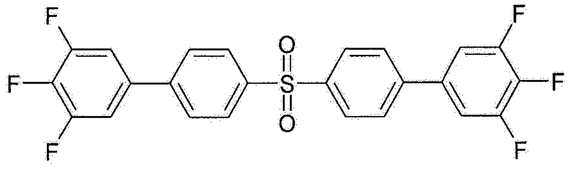

The compound of interest is prepared according to the following scheme.

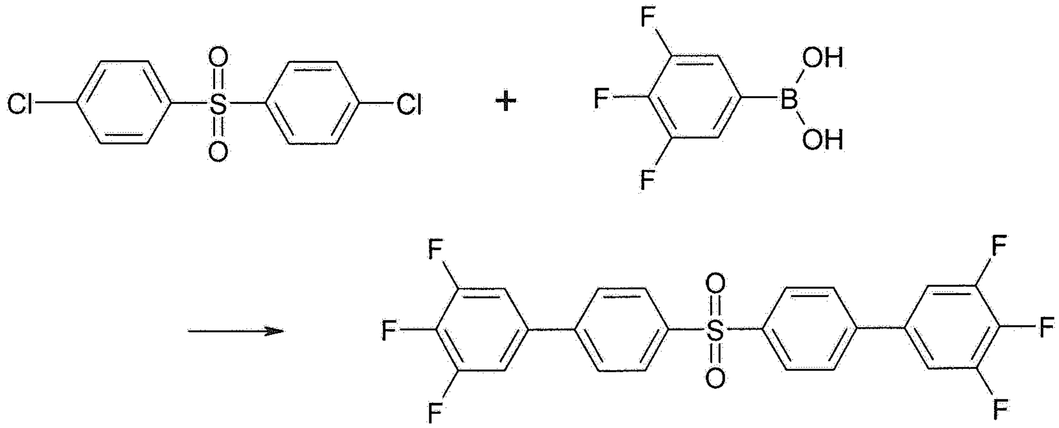

Bis-(4-chlorophenyl)sulphone (2.87 g, 10 mmol, 3,4,5-trifluorobenzene boronic acid (4.22 g, 24 mmol), palladium acetate (PdOAc, 448 mg, 2 mmol), 1,3-Bis(diphenylphosphino)propane (dppp, 840 mg, 1 mmol), caesium fluoride (CsF, 6.07 g, 40 mmol) and N 1-methyl-2-pyrollidinone (35 mL) are ultasonicated for 30 minutes. Then the mixture is heated for 16 hours at 100°C and then cooled. Subsequently water (200 mL) is added and the mixture is extracted with ethyl acetate (2 x 100 mL) in a separating funnel. The organic layers are combined and concentrated under reduced pressure to give a dark coloured solid. This is dissolved in DCM (10 mL) and applied to a column of silica eluted with the following mixtures of petrol 2 : DCM 2:1, 1:1, 1 : 2 and finally pure DCM until all product is eluted. The appropriate fractions are combined and concentrated to give a solid which is re-crystallised from acetonitrile.

The product has the following properties. Phase sequence: K 204 I, e/K = 1.79 $V^{-1}$.

The following compound(s) of formula I are prepared analogously.

Compound 3

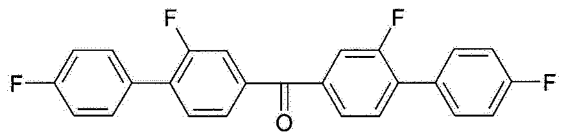

Phase sequence: K 147.8 I, e/K to be determined.

The materials in the above table generally show increased performance in the screening mixtures, as compared to known, more conventional bimesogenic compounds as e.g. those shown in the table below.

Comparative Compound Example 1

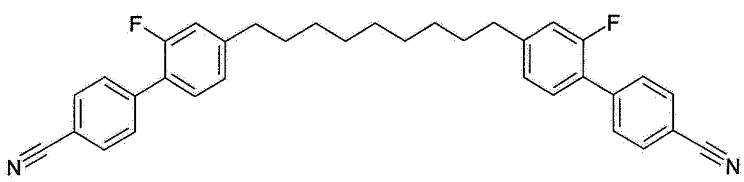

Phase sequence: K 98 (N 83) I, e/K = 2.25 $V^{-1}$.

Use Examples, Mixture Examples

Typically a 5.6 μm thick cell, having an anti-parallel rubbed PI alignment layer, is filled on a hotplate at a temperature at which the flexoelectric mixture in the isotropic phase.

After the cell has been filled phase transitions, including clearing point, are measured using Differential Scanning

Calorimetry (DSC) and verified by optical inspection. For optical phase transition measurements, a Mettler FP90 hot-stage controller connected to a FP82 hot-stage is used to control the temperature of the cell. The temperature is increased from ambient temperature at a rate of 5 degrees C per minute, until the onset of the isotropic phase is observed. The texture change is observed through crossed polarisers using an Olympus BX51 microscope and the respective temperature noted.
Wires are then attached to the ITO electrodes of the cell using indium metal. The cell is secured in a Linkam THMS600 hot-stage connected to a Linkam TMS93 hot-stage controller. The hot-stage is secured to a rotation stage in an Olympus BX51 microscope.

The cell is heated until the liquid crystal is completely isotropic. The cell is then cooled under an applied electric field until the sample is completely nematic. The driving waveform is supplied by a Tektronix AFG3021B arbitrary function generator, which is sent through a Newtons4th LPA400 power amplifier before being applied to the cell. The cell response is monitored with a Thorlabs PDA55 photodiode. Both input waveforms and optical response are measured using a Tektronix TDS 2024B digital oscilloscope.

In order to measure the flexoelastic response of the material, the change in the size of the tilt of the optic axis is measured as a function of increasing voltage. This is achieved by using the equation:

$$\tan\varphi = \frac{P_0}{2\pi}\frac{e}{K}\underline{E}$$

wherein φ is the tilt in the optic axis from the original position (i.e. when E = 0), E is the applied field, K is the elastic constant (average of $K_1$ and $K_3$) and e is the flexoelectric coefficient (where $e = e_1 + e_3$). The applied field is monitored using a HP 34401A multimeter. The tilt angle is measured using the aforementioned microscope and oscilloscope. The undisturbed cholesteric pitch, $P_0$, is measured using an Ocean Optics USB4000 spectrometer attached to a computer. The selective reflection band is obtained and the pitch determined from the spectral data.

The mixtures shown in the following examples are well suitable for use in USH-displays. To that end an appropriate concentration of the chiral dopant or dopants used has to be applied in order to achieve a cholesteric pitch of 200 nm or less.

Comparative Mixture Example 1

Host Mixture H-0

The host mixture H-0 is prepared and investigated.

| Composition | | |
|---|---|---|
| Compound | | |
| No. | Abbreviation | Conc. /% |
| 1 | F-PGI-O-9-O-GP-F | 25.0 |
| 2 | F-PGI-O-9-O-PP-N | 25.0 |
| 3 | F-PGI-ZI-9-Z-GP-F | 25.0 |
| 4 | F-PGI-ZI-9-Z-PP-N | 25.0 |
| Σ | | 100.0 |

2 % of the chiral dopant R-5011 are added to the mixture H-0 leading to the mixture C-1, which is investigated for its properties.

| Composition | | |
|---|---|---|
| Compound | | |
| No. | Abbreviation | Conc. /% |
| 1 | R-5011 | 2.0 |
| 2 | F-PGI-O-9-O-GP-F | 24.5 |
| 3 | F-PGI-O-9-O-PP-N | 24.5 |

(continued)

| Composition | | |
|---|---|---|
| Compound | | |
| No. | Abbreviation | Conc. /% |
| 4 | F-PGI-ZI-9-Z-GP-F | 24.5 |
| 5 | F-PGI-ZI-9-Z-PP-N | 24.5 |
| Σ | | 100.0 |

The mixture C-1 may be used for the ULH-mode. It has a clearing point of 82°C and a lower transition temperature [T(N2,N)] of 33°C. It has a cholesteric pitch of 301 nm at 35°C. The e/K of this mixture is 1.9 $Cm^{-1} \cdot N^{-1}$ at a temperature of 34.8°C.

Mixture Example 1: Mixture M-1

2 % of the chiral dopant R-5011 and 10 % of compound 1 are added to the mixture H-0 leading to the mixture M-1, which is investigated for its properties.

| Composition | | |
|---|---|---|
| Compound | | |
| No. | Abbreviation | Conc. /% |
| 1 | R-5011 | 2.0 |
| 2 | F-PGI-O-9-O-GP-F | 22.0 |
| 3 | F-PGI-O-9-O-PP-N | 22.0 |
| 4 | F-PGI-ZI-9-Z-GP-F | 22.0 |
| 5 | F-PGI-ZI-9-Z-PP-N | 22.0 |
| 6 | Compound 1 | 10.0 |
| Σ | | 100.0 |

This mixture (M-1) is well suitable for the USH-mode and for the ULH-mode. It has a cholesteric pitch of 322 nm at 30.8°C. The e/K of this mixture is 1.97 $Cm^{-1} \cdot N^{-1}$, i.e. 1.97 $V^{-1} \cdot$, at a temperature of 49.8°C.

Mixture Example 2

2 % of the chiral dopant R-5011 and 10 % of compound 2 are added to the mixture H-0 leading to the mixture M-2, which is prepared and investigated for its properties.

| Composition | | |
|---|---|---|
| Compound | | |
| No. | Abbreviation | Conc. /% |
| 1 | R-5011 | 2.0 |
| 2 | F-PGI-O-9-O-GP-F | 22.0 |
| 3 | F-PGI-O-9-O-PP-N | 22.0 |
| 4 | F-PGI-ZI-9-Z-GP-F | 22.0 |
| 5 | F-PGI-ZI-9-Z-PP-N | 22.0 |
| 6 | Compound 2 | 10.0 |
| Σ | | 100.0 |

This mixture (M-2) is well suitable for the USH-mode and for the ULH-mode. The e/K of this mixture is 1.79 $Cm^{-1} \cdot N^{-1}$.

Mixture Example 3: Mixture M-3

2 % of the chiral dopant R-5011 and 10 % of compound 3 are added to the mixture H-0 leading to the mixture M-3, which is prepared and investigated for its properties.

Mixture M-3

| Composition | | |
|---|---|---|
| Compound | | |
| No. | Abbreviation | Conc. /% |
| 1 | R-5011 | 2.0 |
| 2 | F-PGI-O-9-O-GP-F | 22.0 |
| 3 | F-PGI-O-9-O-PP-N | 22.0 |
| 4 | F-PGI-ZI-9-Z-GP-F | 22.0 |
| 5 | F-PGI-ZI-9-Z-PP-N | 22.0 |
| 6 | Compound 3 | 10.0 |
| Σ | | 100.0 |

This mixture (M-3) is well suitable for the USH-mode and for the ULH-mode.

## Claims

1. Liquid-crystalline medium, **characterised in that** it comprises one or more bimesogenic compounds of formula I

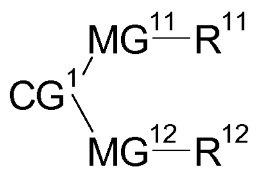

I

in which

$R^{11}$ and $R^{12}$ are each independently $OCF_3$, $CF_3$, F, Cl or CN,
$MG^{11}$ and $MG^{12}$ are each independently a mesogenic group,
wherein at least one of
$MG^{11}$ and $MG^{12}$ comprises one, two or more 5-atomic and/or 6-atomic rings, in case of comprising two or more 5- and/or 6-atomic rings at least two of these may be linked by a 2-atomic linking group, and
$CG^1$ is a central atom or a central group having a total length of one atom,

and
one or more compounds selected from the group of compounds of formula III

$$R^{31}\text{-}MG^{31}\text{-}X^{31}\text{-}Sp^{3}\text{-}X^{32}\text{-}MG^{32}\text{-}R^{32} \qquad \text{III}$$

wherein

$R^{31}$ and $R^{32}$ are each independently H, F, Cl, CN, NCS or a straight-chain or branched alkyl group with 1 to 25 C atoms which may be unsubstituted, mono- or polysubstituted by halogen or CN, it being also possible for one or more non-adjacent $CH_2$ groups to be replaced, in each case independently from one another, by -O-, -S-, -NH-, -N($CH_3$)-, -CO-, -COO-, -OCO-, -O-CO-O-, -S-CO-, -CO-S-, -CH=CH-, -CH=CF-, -CF=CF- or -C≡C- in

such a manner that oxygen atoms are not linked directly to one another,

$MG^{31}$ and $MG^{32}$ are each independently a mesogenic group,

$Sp^3$ is a spacer group comprising 5 to 40 C atoms, wherein one or more non-adjacent $CH_2$ groups may also be replaced by -O-, -S-, -NH-, -N($CH_3$)-, -CO-, -O-CO-, -S-CO-, -O-COO-, -CO-S-, -CO-O-, -CH(halogen)-, -CH(CN)-, -CH=CH- or -C≡C-, and

$X^{31}$ and $X^{32}$ are each independently -O-, -S-, -CO-, -COO-, -OCO-, -O-CO-O-, -CO-NH-, -NH-CO-, -$CH_2CH_2$-, -$OCH_2$-, -$CH_2O$-, -$SCH_2$-, -$CH_2S$-, -CH=CH-, -CH=CH-COO-, -OCO-CH=CH-, -C≡C- or a single bond, and

with the condition that compounds of formula I are excluded.

**2.** Liquid-crystalline medium according to claim 1, wherein in formula I the group

$CG^1$ is selected from -$CH_2$-, -CHF-, -$CF_2$-, -O-, -S-, -(C=O)-, -CH(OR)-, -CH(R)-, -C(R)(R')-, -$SO_2$-, -CH($CF_3$)-, -C(=$CH_2$)-, -NH-, -N(R)- and -S(R)(R')-,

R and R' are independently of each other an alkyl group having 1 to 5 carbon atoms, wherein one or two non-terminal and non-adjacent -$CH_2$- groups may be replaced by O.

**3.** Liquid-crystalline medium according to claim 1 or 2, wherein in formula I the group $CG^1$ is selected from -$CH_2$-, -$CF_2$-, -O-, -CO- and -$SO_2$-.

**4.** Liquid-crystalline medium according to one or more of claims 1 to 3, wherein the medium comprises one or more compounds of formula I of claim 1 in which $MG^{11}$ and $MG^{12}$ each, identically or differently, are selected from the following formulae IIa to IIn and their mirror images

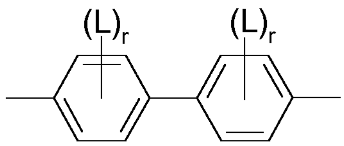

IIa

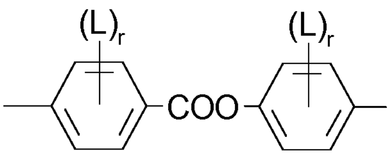

IIb

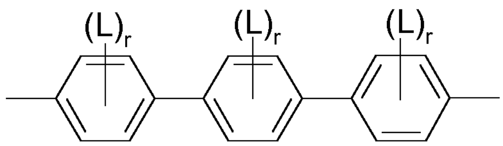

IIc

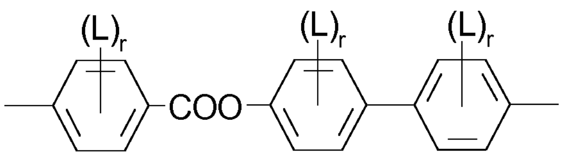

IId

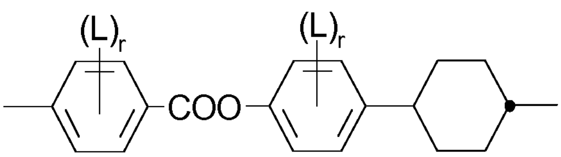

IIe

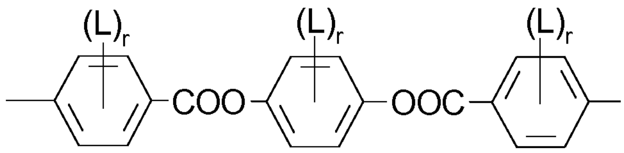
IIf

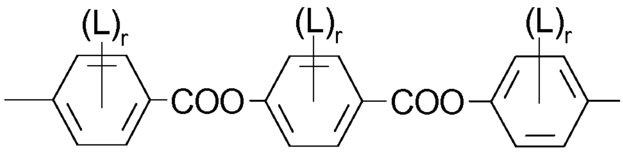
IIg

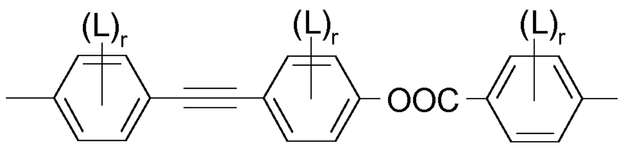
IIh

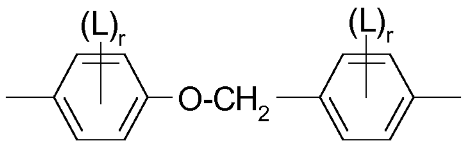
II i

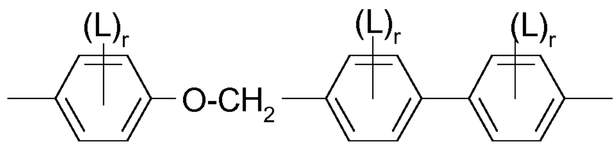
II j

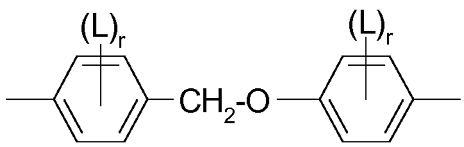
IIk

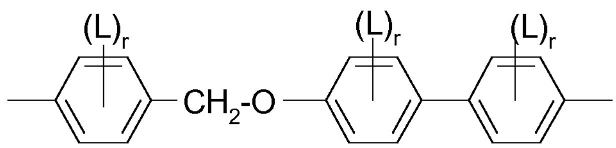
II l

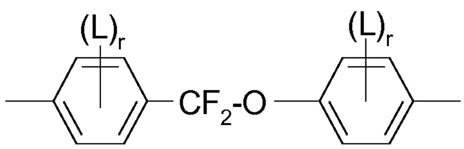
IIm

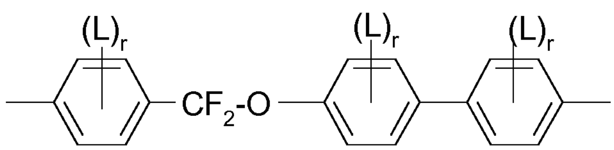
IIn

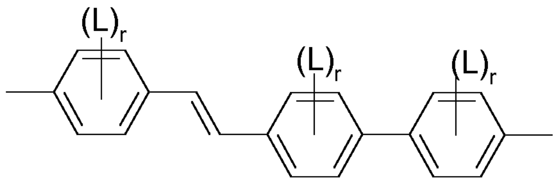

IIo

in which

L on each occurence, identically or differently, denotes F or Cl, and
r on each occurrence, identically or differently, is 0, 1, 2 or 3.

**5.** Liquid-crystalline medium according to one or more of claims 1 to 4, wherein the medium comprises one or more compounds of the following formulae:

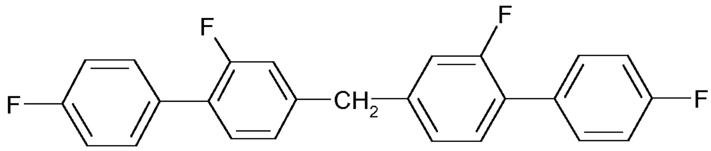

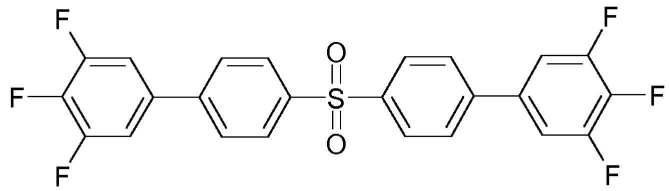

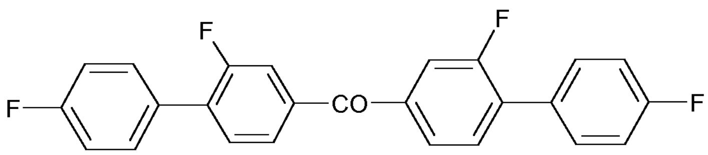

.

**6.** Use of a liquid crystal medium according to one or more of claims claim 1 to 5 in a liquid crystal device.

**7.** Liquid crystal device comprising a liquid-crystalline medium according to one or more of claims 1 to 6.

**8.** Liquid crystal device according to claim 7, **characterized in that** it is a flexoelectric device.

**9.** Preparation of a liquid crystal medium according to one or more of claims 1 to 5, **characterized in that** one or more compounds of formula I according to one or more of claims 1 to 5 is mixed with one or more compounds of formula III, as defined in claim 1 and/or one or more further compounds.

**Patentansprüche**

**1.** Flüssigkristallines Medium, **dadurch gekennzeichnet, dass** es eine oder mehrere bimesogene Verbindungen der Formel I

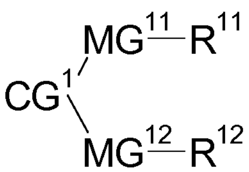

I

bei der

$R^{11}$ und $R^{12}$ jeweils unabhängig $OCF_3$, $CF_3$, F, Cl oder CN sind,
$MG^{11}$ und $MG^{12}$ jeweils unabhängig eine mesogene Gruppe sind,

bei denen mindestens einer von

$MG^{11}$ und $MG^{12}$ einen, zwei oder mehr 5-atomige und/oder 6-atomige Ringe enthält, wobei bei zwei oder mehr enthaltenen 5- und/oder 6-atomigen Ringen mindestens zwei davon durch eine 2-atomige Verknüpfungsgruppe verknüpft sein können, und
$CG^1$ ein Zentralatom oder eine zentrale Gruppe mit einer Gesamtlänge von einem Atom ist,

und
eine oder mehrere Verbindungen, die aus der Gruppe der Verbindungen der Formel III

$$R^{31}\text{-}MG^{31}\text{-}X^{31}\text{-}Sp^{3}\text{-}X^{32}\text{-}MG^{32}\text{-}R^{32} \qquad \text{III}$$

ausgewählt sind, bei der

$R^{31}$ und $R^{32}$ jeweils unabhängig H, F, Cl, CN, NCS oder eine geradkettige oder verzweigte Alkylgruppe mit 1 bis 25 C-Atomen sind, die unsubstituiert oder ein- oder mehrfach durch Halogen oder CN substituiert sein kann, wobei auch eine oder mehrere nicht benachbarte $CH_2$-Gruppen jeweils unabhängig voneinander so durch -O-, -S-, -NH-, -N($CH_3$)-, -CO-, -COO-, -OCO-, -O-CO-O-, -S-CO-, -CO-S-, -CH=CH-, -CH=CF-, -CF=CF- oder -C≡C- ersetzt sein können, dass Sauerstoffatome nicht direkt miteinander verknüpft sind,
$MG^{31}$ und $MG^{32}$ jeweils unabhängig eine mesogene Gruppe sind,
$Sp^3$ eine Spacergruppe mit 5 bis 40 C-Atomen ist, bei der auch eine oder mehrere nicht benachbarte $CH_2$-Gruppen durch -O-, -S-, -NH-, -N($CH_3$)-, -CO-, -O-CO-, -S-CO-, -O-COO-, -CO-S-, -CO-O-, -CH(Halogen)-, -CH(CN)-, -CH=CH- oder -C≡C- ersetzt sein können und
$X^{31}$ und $X^{32}$ jeweils unabhängig -O-, -S-, -CO-, -COO-, -OCO-, -O-CO-O-, -CO-NH-, -NH-CO-, -$CH_2CH_2$-, -$OCH_2$-, -$CH_2O$-, -$SCH_2$-, -$CH_2S$-, -CH=CH-, -CH=CH-COO-, -OCO-CH=CH-, -C≡C- oder eine Einfachbindung sind und

unter der Bedingung, dass Verbindungen der Formel I ausgenommen sind,
enthält.

**2.** Flüssigkristallines Medium nach Anspruch 1, bei dem in Formel I die Gruppe

$CG^1$ aus -$CH_2$-, -CHF-, -$CF_2$-, -O-, -S-, -(C=O)-, -CH(OR)-, -CH(R)-, -C(R)(R')-, -$SO_2$-, -CH($CF_3$)-, -C(=$CH_2$)-, -NH-, -N(R)- und -S(R)(R')- ausgewählt ist,
R und R' unabhängig voneinander eine Alkylgruppe mit 1 bis 5 Kohlenstoffatomen sind, bei der eine oder zwei nicht endständige und nicht benachbarte -$CH_2$-Gruppen durch O ersetzt sein können.

**3.** Flüssigkristallines Medium nach Anspruch 1 oder 2, bei dem in Formel I die Gruppe $CG^1$ aus -$CH_2$-, -$CF_2$-, -O-, -CO- und -$SO_2$- ausgewählt sind.

**4.** Flüssigkristallines Medium nach einem oder mehreren der Ansprüche 1 bis 3, bei dem das Medium eine oder mehrere Verbindungen der Formel I des Anspruchs 1 enthält, in der $MG^{11}$ und $MG^{12}$ jeweils gleich oder verschieden aus den folgenden Formeln IIa bis IIn und ihren Spiegelbildern

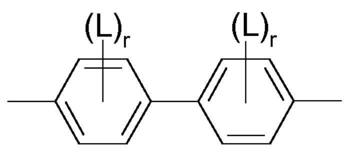

IIa

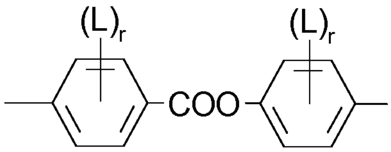

IIb

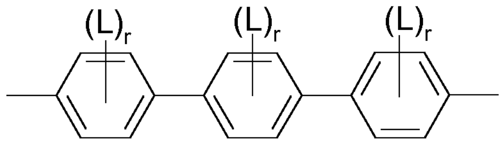

IIc

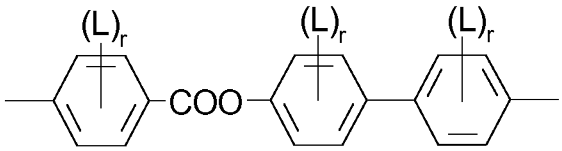

IId

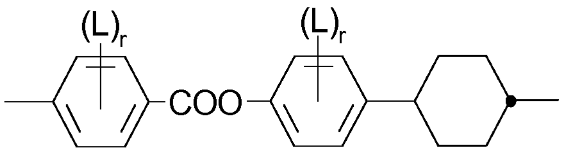

IIe

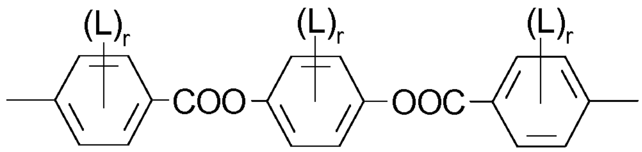

IIf

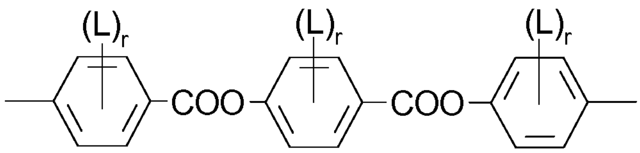

IIg

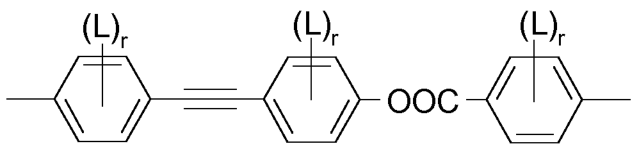

IIh

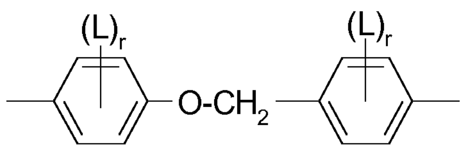

II i

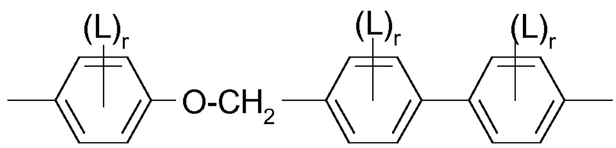

II j

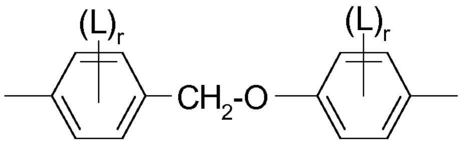

IIk

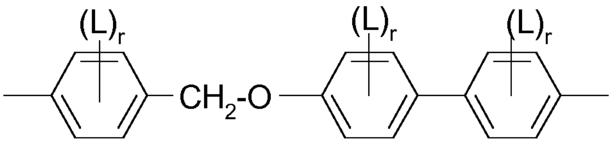

II l

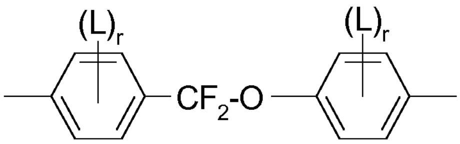

IIm

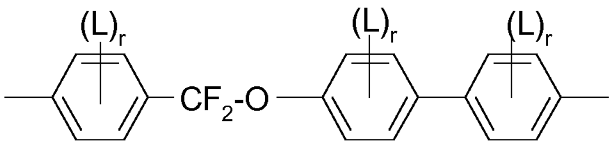

IIn

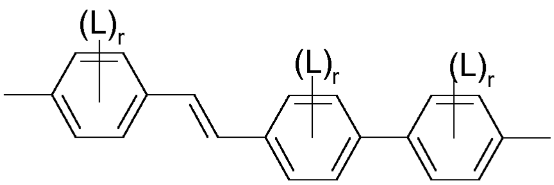

IIo

ausgewählt sind, worin

L bei jedem Auftreten gleich oder verschieden F oder Cl bedeutet und
r bei jedem Auftreten gleich oder verschieden 0, 1, 2 oder 3 ist.

**5.** Flüssigkristallines Medium nach einem oder mehreren der Ansprüche 1 bis 4, bei dem das Medium eine oder mehrere Verbindungen der folgenden Formeln enthält:

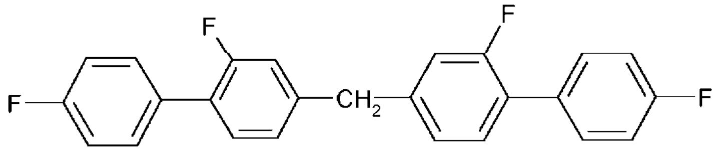

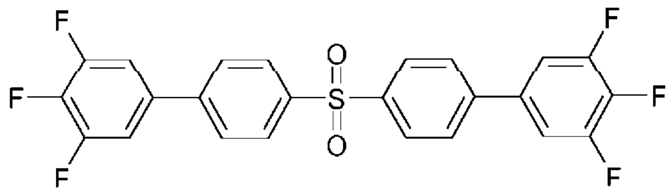

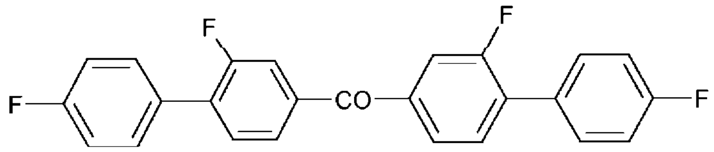

6. Verwendung eines Flüssigkristallmediums nach einem oder mehreren der Ansprüche 1 bis 5 in einer Flüssigkristallvorrichtung.

7. Flüssigkristallvorrichtung enthaltend ein flüssigkristallines Medium nach einem oder mehreren der Ansprüche 1 bis 6.

8. Flüssigkristallvorrichtung nach Anspruch 7, **dadurch gekennzeichnet, dass** es sich um eine flexoelektrische Vorrichtung handelt.

9. Herstellung eines Flüssigkristallmediums nach einem oder mehreren der Ansprüche 1 bis 5, **dadurch gekennzeichnet, dass** man eine oder mehrere Verbindungen der Formel I nach einem oder mehreren der Ansprüche 1 bis 5 mit einer oder mehreren Verbindungen der Formel III, wie in Anspruch 1 definiert, und/oder einer oder mehreren weiteren Verbindungen mischt.

**Revendications**

1. Milieu cristallin liquide, **caractérisé en ce qu'**il comprend :

un ou plusieurs composé(s) bimésogène(s) de la formule I :

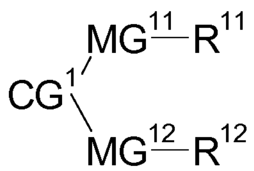

I

dans laquelle :

$R^{11}$ et $R^{12}$ sont, chacun de manière indépendante, $OCF_3$, $CF_3$, F, Cl ou CN ;
$MG^{11}$ et $MG^{12}$ sont, chacun de manière indépendante, un groupe mésogène ;

où au moins l'un de

$MG^{11}$ et $MG^{12}$ comprend un cycle, deux cycles ou plus de deux cycles à 5 atomes et/ou à 6 atomes, dans le cas où il comprend deux cycles ou plus de deux cycles à 5 atomes et/ou à 6 atomes, au moins deux de ces cycles peuvent être liés au moyen d'un groupe de liaison à 2 atomes ; et
$CG^1$ est un atome central ou un groupe central qui présente une longueur totale d'un atome ;

et
un ou plusieurs composé(s) qui est/sont sélectionné(s) parmi le groupe de composés de la formule III :

$$R^{31}\text{-}MG^{31}\text{-}X^{31}\text{-}Sp^{3}\text{-}X^{32}\text{-}MG^{32}\text{-}R^{32} \qquad \text{III}$$

dans laquelle :

$R^{31}$ et $R^{32}$ sont, chacun de manière indépendante, H, F, Cl, CN, NCS ou un groupe alkyle en chaîne droite ou ramifié qui comporte de 1 à 25 atome(s) de C, lequel peut être non substitué, mono- ou polysubstitué par halogène ou par CN, étant entendu qu'il est également possible qu'un ou que plusieurs groupe(s) $CH_2$ non adjacents soit/soient remplacé(s), dans chaque cas de manière indépendante les uns des autres, par -O-, -S-, -NH-, $-N(CH_3)-$, -CO-, -COO-, -OCO-, -O-CO-O-, -S-CO-, -CO-S-, -CH=CH-, -CH=CF-, -CF=CF-

ou -C≡C- de telle sorte que des atomes d'oxygène ne soient pas liés directement les uns aux autres ;
$MG^{31}$ et $MG^{32}$ sont, chacun de manière indépendante, un groupe mésogène,
$Sp^3$ est un groupe d'espaceur qui comprend de 5 à 40 atomes de C, dans lequel un ou plusieurs groupe(s) $CH_2$ non adjacents peut/peuvent également être remplacé(s) par -O-, -S-, -NH-, -N($CH_3$)-, -CO-, -O-CO-, -S-CO-, -O-COO-, -CO-S-, -CO-O-, -CH(halogène)-, -CH(CN)-, -CH=CH- ou -C≡C- ; et
$X^{31}$ et $X^{32}$ sont, chacun de manière indépendante, -O-, -S-, -CO-, -COO-, -OCO-, -O-CO-O-, -CO-NH-, -NH-CO-, -$CH_2CH_2$-, -$OCH_2$-, -$CH_2O$-, -$SCH_2$-, -$CH_2S$-, -CH=CH-, -CH=CH-COO-, -OCO-CH=CH-, -C≡C- ou une liaison simple ; et

moyennant la condition consistant **en ce que** les composés de la formule I sont exclus.

**2.** Milieu cristallin liquide selon la revendication 1, dans lequel, dans la formule I, le groupe

$CG^1$ est sélectionné parmi -$CH_2$-, -CHF-, -$CF_2$-, -O-, -S-, -(C=O)-, -CH(OR)-, -CH(R)-, -C(R)(R')-, -$SO_2$-, -CH($CF_3$)-, -C(=$CH_2$)-, -NH-, -N(R)- et -S(R)(R')- ;
R et R' sont, de manière indépendante de l'autre, un groupe alkyle qui comporte de 1 à 5 atome(s) de carbone, dans lequel un ou deux groupe(s) -$CH_2$- non terminal/ terminaux et non adjacents peut/peuvent être remplacé(s) par O.

**3.** Milieu cristallin liquide selon la revendication 1 ou 2, dans lequel, dans la formule I, le groupe $CG^1$ est sélectionné parmi -$CH_2$-, -$CF_2$-, -O-, -CO- et -$SO_2$-.

**4.** Milieu cristallin liquide selon une ou plusieurs des revendications 1 à 3, dans lequel le milieu comprend un ou plusieurs composé(s) de la formule I selon la revendication 1 dans laquelle $MG^{11}$ et $MG^{12}$ sont chacun, de manière identique ou différente, sélectionnés parmi les formules IIa à IIn qui suivent et leurs images miroir :

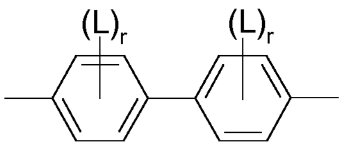
IIa

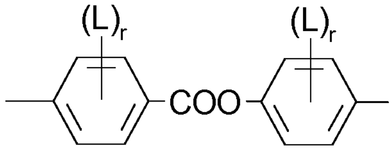
IIb

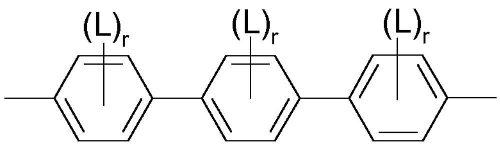
IIc

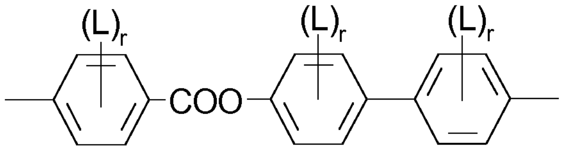
IId

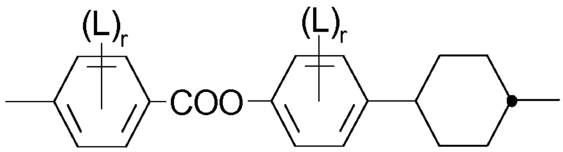
IIe

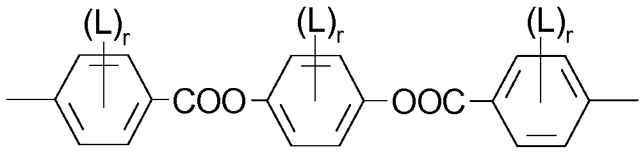

IIf

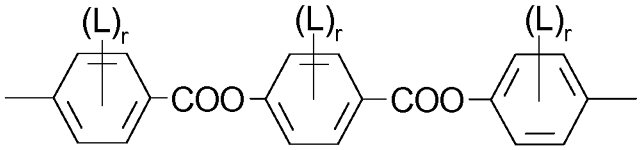

IIg

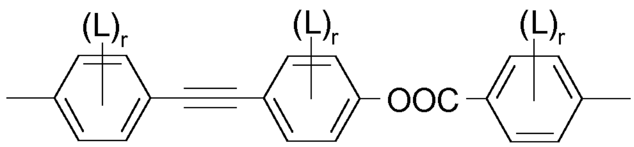

IIh

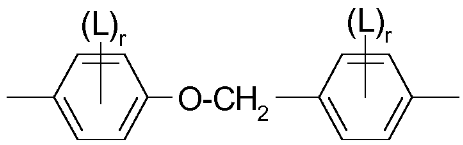

II i

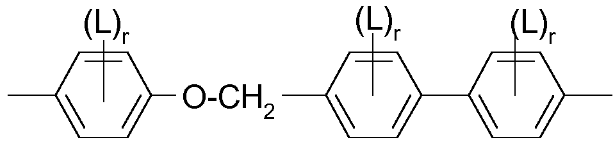

II j

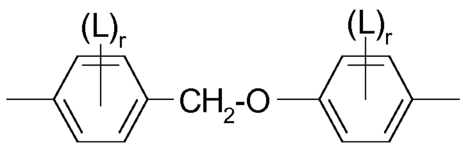

IIk

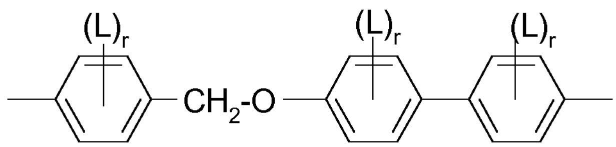

II l

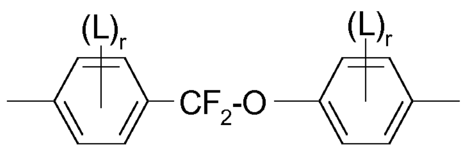

IIm

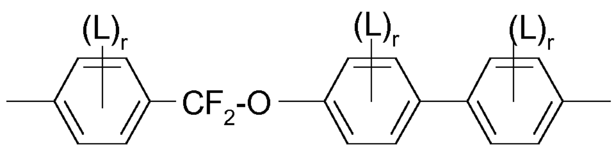

IIn

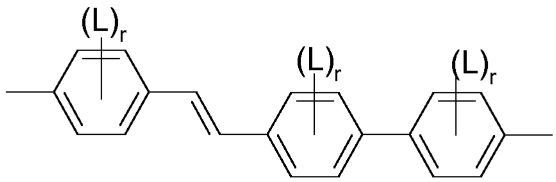

IIo

dans lesquelles :

L représente, pour chaque occurrence, de manière identique ou différente, F ou Cl ; et
r est, pour chaque occurrence, de manière identique ou différente, 0, 1, 2 ou 3.

**5.** Milieu cristallin liquide selon une ou plusieurs des revendications 1 à 4, dans lequel le milieu comprend un ou plusieurs composé(s) des formules qui suivent :

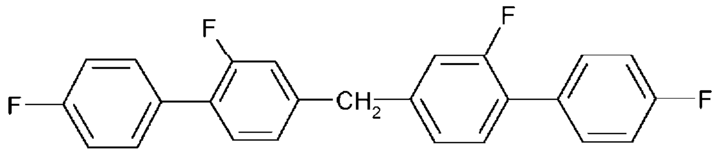

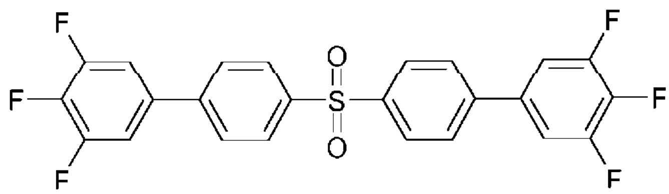

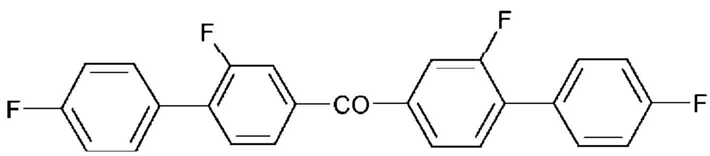

**6.** Utilisation d'un milieu cristallin liquide selon une ou plusieurs des revendications 1 à 5 dans un dispositif à cristaux liquides.

**7.** Dispositif à cristaux liquides, comprenant un milieu cristallin liquide selon une ou plusieurs des revendications 1 à 6.

**8.** Dispositif à cristaux liquides selon la revendication 7, **caractérisé en ce qu'**il s'agit d'un dispositif flexoélectrique.

**9.** Préparation d'un milieu cristallin liquide selon une ou plusieurs des revendications 1 à 5, **caractérisée en ce qu'**un ou plusieurs composé(s) de la formule I selon une ou plusieurs des revendications 1 à 5 est/sont mélangé(s) avec un ou plusieurs composé(s) de la formule III, comme défini selon la revendication 1 et/ou avec un ou plusieurs autre(s) composé(s).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4000451
- EP 0588568 A
- EP 0971016 A
- GB 2356629 A
- WO 2014005672 A
- EP 0233688 A
- CN 102617304
- JP 2006206464 A
- WO 2006061094 A1
- WO 9800428 A
- GB 2328207 A


### Non-patent literature cited in the description


- **CHANDRASEKHAR.** Liquid Crystals. Cambridge University Press, 1992
- **P.G. DEGENNES et al.** The Physics of Liquid Crystals. Oxford Science Publications, 1995
- **HORI, K. ; LIMURO, M. ; NAKAO, A. ; TORIUMI, H.** *J. Mol. Struc.,* 2004, vol. 699, 23-29
- **H.J. COLES et al.** *J. Appl. Phys.,* 2006, vol. 99, 034104
- **COLES, H.J. ; MUSGRAVE, B. ; COLES, M.J. ; WILLMOTT, J.** *J. Mater. Chem.,* 2001, vol. 11, 2709-2716
- Polynuclear Paracyclophanes. **MACRO RINGS. 11.** JACS. 1952, vol. 74, 5388-91
- *Molecular Crystals and Liquid Crystals,* 1984, vol. 102 (8-9), 223-233
- *Tetrahedron,* vol. 27 (36), 4331-4334
- New mesogens with a fluorosubstituted bent core. **MAHAJAN R et al.** Liquid Crystals. Taylor & Francis, 01 February 2004, vol. 31, 161-167
- **BARNES, P.J. ; DOUGLAS, A.G. ; HEEKS, S.K. ; LUCKHURST, G.R.** *Liquid Crystals,* 1993, vol. 13 (4), 603-613
- **HENDERSON, P.A. ; NIEMEYER, O. ; IMRIE, C.T.** *Liquid Crystals,* 2001, vol. 28 (3), 463-472
- **HENDERSON, P.A. ; SEDDON, J.M. ; IMRIE, C.T.** *Liquid Crystals,* 2005, vol. 32 (11-12), 1499-1513
- **PANOV, V.P. ; NGARAJ, M. ; VIJ, J.K. ; PANARIN, Y.P. ; KOHLMEIER, A. ; TAMBA, M.G. ; LEWIS, R.A. ; MEHL, G.H.** *Phys.Rev.Lett.,* 2010, vol. 105, 1678011-1678014
- **P. RUDQUIST et al.** *Liq. Cryst.,* 1997, vol. 23 (4), 503
- **J. COGNARD.** *Mol. Cryst. Liq. Cryst.,* 1981, vol. 78 (1), 1-77